# FASCICULE DE BREVET EUROPEEN

(11) **EP 2 220 254 B1**
(45) Date de publication et mention de la délivrance du brevet: **03.10.2012**
(21) Numéro de dépôt: 08865683.0
(22) Date de dépôt: 04.12.2008
(51) Int. Cl.: C12Q 1/68

(54) **NOUVEAU PROCÉDÉ DE GÉNÉRATION ET DE CRIBLAGE D'UNE BANQUE D'ANTICORPS**
NEUES VERFAHREN ZUR ERSTELLUNG UND DURCHSICHT EINER ANTIKÖRPERBIBLIOTHEK
NOVEL METHOD FOR GENERATING AND SCREENING AN ANTIBODY LIBRARY

(30) Priorité: 04.12.2007 FR 0708444
(43) Date de publication de la demande: 25.08.2010
(73) Titulaire: Pierre Fabre Medicament, 92100 Boulogne-Billancourt (FR)
(72) Inventeur: LOWE, Peter, F-74100 Ambilly (FR); BES, Cédric, F-30250 Villevieille (FR); BOUTE, Nicolas, F-74350 Cernex (FR)
(74) Mandataire: Faivre Petit, Frédérique
(86) Numéro de dépôt international: PCT/EP2008/066804
(87) Numéro de publication internationale: WO 2009/080461

(56) Documents cités:
- WO-A-01/79481
- WO-A-03/054016
- WO-A-2006/063355
- WO-A-2008/012529
- US-B2- 6 635 424
- LOH E Y ET AL: "POLYMERASE CHAIN REACTION WITH SINGLE-SIDED SPECIFICITY: ANALYSIS OF T CELL RECEPTOR DELTA CHAIN" SCIENCE, WASHINGTON, DC, vol. 243, no. 4888, 13 janvier 1989 (1989-01-13), pages 217-220, XP000673517 ISSN: 0036-8075
- POLIDOROS ALEXIOS N ET AL: "Rolling circle amplification-RACE: a method for simultaneous isolation of 5' and 3' cDNA ends from amplified cDNA templates." BIOTECHNIQUES JUL 2006, vol. 41, no. 1, juillet 2006 (2006-07), pages 35-36 , 38 ,, XP002488782 ISSN: 0736-6205 cité dans la demande
- CHRIST DANIEL ET AL: "Tapping diversity lost in transformations - in vitro amplification of ligation reactions" NUCLEIC ACIDS RESEARCH, OXFORD UNIVERSITY PRESS, SURREY, GB, vol. 34, no. 16, 1 septembre 2006 (2006-09-01), XP002461598 ISSN: 0305-1048

## Description

La présente invention concerne le domaine des anticorps monoclonaux, notamment humains. Plus particulièrement, la présente invention vise un nouveau procédé de génération d'une séquence d'ADN codant pour une chaîne lourde et/ou une chaîne légère d'au moins un anticorps, de préférence à partir d'ARN total ou messager issu de cellule capable d'exprimer un anticorps. L'invention comprend également un procédé de génération d'une banque ou librairie d'anticorps, préférentiellement humains, mettant en oeuvre les étapes du procédé précédent.

Les expressions « banque(s) » ou « librairie(s) » seront utilisées indifféremment dans la présente description, ces deux expressions ayant le même sens.

Traditionnellement, les anticorps monoclonaux, ou les fragments de ceux-ci, étaient préparés en utilisant la technique classique des hybridomes décrites par Kohler et Milstein (Kohler et Milstein, 1975, Nature 256, 495), les anticorps ainsi obtenus étant ensuite humanisés.

Plus récemment, de nouvelles méthodes, basées sur la compréhension des mécanismes naturels et sur l'évolution des techniques de recombinaison d'ADN, ont été développées pour générer et exprimer des anticorps monoclonaux humains. En particulier, les techniques visant à obtenir des librairies, ou banques, combinatoires d'anticorps ont fait l'objet de nombreux développements. De telles techniques, outre le fait d'être plus rapides et de permettre de s'affranchir des étapes de l'humanisation, permettent d'utiliser plus efficacement l'ensemble et la variabilité du répertoire d'anticorps.

Les anticorps sont des glycoprotéines de la superfamille des immunoglobulines formées de 4 chaînes polypeptidiques : 2 chaînes lourdes (H pour *heavy*) et 2 chaînes légères (L pour *light)* qui sont reliées entre elles par un nombre variable de ponts disulfures assurant une flexibilité à la molécule. Ces chaînes forment une structure en Y et sont constituées de domaines immunoglobulines de 110 acides aminés environ. Chaque chaîne légère est constituée d'une région constante et d'une région variable alors que les chaînes lourdes sont composées d'une région variable et de 3 ou 4 régions constantes selon l'isotype.

Pour un anticorps donné, les deux chaînes lourdes sont identiques, de même pour les deux chaînes légères.

Les régions constantes sont caractérisées par des séquences en acides aminés très proches d'un anticorps à l'autre, et sont caractéristiques de l'espèce et de l'isotype. Chaque chaîne légère en possède un exemplaire noté C_{L}. Les chaînes lourdes comportent, selon l'isotype, trois ou quatre régions constantes C_{H}1, C_{H}2, C_{H}3 et C_{H}4.

Un anticorps possède quatre régions variables situées aux extrémités des deux « bras ». L'association entre une région variable portée par une chaîne lourde (V_{H}) et la région variable adjacente portée par une chaîne légère (V_{L}) constitue le site de reconnaissance (ou paratope) de l'antigène. Ainsi, une molécule d'immunoglobuline possède deux sites de liaison à l'antigène, un au bout de chaque bras. Ces deux sites sont identiques, d'où la possibilité de lier deux molécules d'antigène par anticorps.

Pour les chaînes lourdes, les régions variables V_{H} sont codées par des gènes qui comportent trois types de segments, à savoir un segment de variabilité (segment ou gène v), un segment de diversité (segment ou gène d) et un segment de jonction (segment ou gène j). Les gènes codant pour les régions variables de la chaîne légère V_{L} possèdent également un gène v et un gène j mais aucun gène d.

Initialement, le répertoire immunitaire naïf dérive du réarrangement combinatoire des différents gènes codant pour les régions variables. Plus particulièrement, les gènes v, d, j pour la chaîne lourde et v, j pour la chaîne légère peuvent s'associer de façon indépendante, ce qui explique la grande diversité de régions variables pouvant être générées. Ce premier mécanisme permettant de produire un très grand nombre d'unités fonctionnelles de structures différentes est appelé diversité combinatoire.

Deux autres mécanismes viennent accroître cette diversité, l'un intervenant à l'étape initiale de la différenciation des lymphocytes B (diversité jonctionnelle) et l'autre au cours de la réponse immunitaire induite par stimulation antigénique (hypermutations somatiques).

La diversité jonctionnelle repose sur l'apparition de mutations lors du réarrangement combinatoire au niveau des jonctions entre les gènes v-d et d-j pour la chaîne lourde et v-j pour la chaîne légère.

Une maturation de l'affinité des anticorps est effectuée par un processus de sélection d'hypermutations somatiques qui consistent en des mutations ponctuelles survenant exclusivement dans les régions codant les régions V_{H} et V_{L} et qui peuvent être à l'origine de changements dans la structure du paratope.

La diversité repose donc sur les trois éléments décrits, à savoir la diversité combinatoire, la diversité jonctionnelle et les hymermutations somatiques.

Partant de ces éléments, différentes technologies ont été mises au point pour pouvoir générer rapidement une grande diversité d'anticorps humains et se sont avérées particulièrement intéressantes dans le cas d'anticorps humains pour lesquels les hybridomes sont techniquement difficiles à obtenir.

Plus particulièrement, l'avènement de la technologie dite du « phage display » (Smith, 1985, Science, 228:1315 ; Scott & Smith, 1990, Science, 249:386 ; McCafferty, 1990, Nature, 348:552) a permis de pouvoir sélectionner *in vitro,* à partir de librairies, des anticorps ou fragments d'anticorps humains dirigés contre une grande variété de cibles, ou antigènes. Ces librairies se divisent en deux groupes, à savoir les librairies naturelles et les librairies synthétiques.

Les librairies naturelles sont construites à partir des gènes directement récupérés des cellules B humaines, donc après les différents mécanismes assurant la diversité combinatoire, la diversité jonctionnelle et les hypermutations somatiques. De telles librairies permettent donc de générer naturellement une grande variabilité d'anticorps.

En outre, ces librairies naturelles présentent l'avantage d'utiliser une technologie établie et validée et permettent une compatibilité avec des systèmes d'expression variés comme les bactéries, les levures, les cellules eucaryotes ou les plantes.

Cependant, la génération de ces librairies reste lourde et difficile à mettre en oeuvre et nécessite le recours à une étape de PCR (« Polymerase Chain Reaction »). Pour la mise en oeuvre de cette étape de PCR, il est nécessaire de connaître les séquences respectives des régions 5' et 3' afin de définir, à partir des lignées germinales connues, les amorces nécessaires pour cette PCR. Si ce point n'est pas particulièrement gênant pour l'extrémité 3' qui se situe au niveau d'un gène c (c'est-à-dire codant pour la région constante de l'anticorps), il est particulièrement limitant pour ce qui est de l'extrémité 5' qui se trouve, quant à elle, au niveau de l'extrémité du gène v, c'est-à-dire du gène de variabilité. Cette connaissance nécessaire de la région 5' du gène v limite, de manière inhérente, les amorces sélectionnables aux amorces issues des lignées germinales connues à ce jour et donc aux séquences de gène v connues. Cela a pour effet de limiter les anticorps pouvant être récupérés aux anticorps présentant des séquences connues au niveau de la région 5' de leur région variable, laissant donc de côté tous les autres anticorps dont les séquences des extrémités 5' des gènes v ne seraient pas connues. En outre, il peut être nécessaire de modifier les acides-aminés dans la région variable pour faciliter le clonage et/ou l'amplification.

Un autre inconvénient résultant de la nécessaire connaissance de l'extrémité 5' du gène v est le risque de laisser passer les anticorps pour lesquels des hypermutations somatiques se seraient produites à cette extrémité.

Enfin, de par la technique de la PCR, ne seront récupérés en priorité que les anticorps pour lesquels l'extrémité 5' du gène v présentera une très forte affinité avec les amorces utilisées, les plus faibles affinités n'étant pas retenues.

Les librairies synthétiques sont, quant à elles, construites à partir des différents gènes non réarrangés. Leur réarrangement est réalisé *in vitro* par PCR et, par la suite, différents mécanismes de maturation *in vitro* peuvent être appliqués pour obtenir les meilleurs anticorps en terme de spécificité et d'affinité.

Le recours à des librairies synthétiques permet d'augmenter la diversité des anticorps obtenus et reste compatible avec les diverses systèmes d'expression. Cependant, il n'en reste pas moins vrai que le travail nécessaire à leur génération reste long et laborieux et de nombreux inconvénients persistent. En effet, pour la réalisation de librairies synthétiques, une étape de PCR reste toujours obligatoire. Outre les inconvénients cités plus haut liés à l'utilisation de la PCR, cette étape nécessite l'utilisation d'amorces dégénérées de grande taille qui introduisent fréquemment des délétions, additions et modifications de paires de base au sein des gènes v, d, j assemblés, ce qui peut également aboutir à la formation d'anticorps incapables de se replier correctement et par conséquent non fonctionnels. De plus, les anticorps sélectionnés à partir de ces librairies sont généralement faiblement exprimés et, dans beaucoup de cas, contiennent des mutations pouvant affecter l'immunogénicité de l'anticorps.

De plus, la génération de librairies synthétiques ne permet pas de prendre en compte les mutations au niveau des zones de jonction assurant la diversité jonctionnelle ni les hypermutations somatiques. Il y a donc une grande perte de variabilité.

La présente invention vise à pallier l'ensemble des inconvénients énumérés ci-dessus en décrivant, pour la première fois, un procédé simple permettant de générer une séquence d'ADN codant pour une chaîne lourde et/ou une chaîne légère, et tout particulièrement permettant de générer une librairie d'anticorps présentant une grande diversité. L'invention a ainsi pour objet un procédé de génération d'une librairie naturelle de chaînes lourdes et/ou chaînes légères d'anticorps présentant non seulement ses avantages mais également ceux d'une librairie synthétique, tout en s'affranchissant des inconvénients respectifs de chacune des deux technologies.

Plus particulièrement, la présente invention se différencie de l'ensemble des techniques connues à ce jour en s'affranchissant de l'étape d'amplification par PCR et, de ce fait, de l'ensemble des inconvénients décrits plus haut liés à cette étape de PCR. Tout particulièrement, la technologie objet de la présente invention ne nécessite plus de connaître la séquence de l'extrémité 5' du gène v du domaine variable de l'anticorps. La présente invention se différencie de l'art antérieur par l'étape d'amplification qui est réalisée sur une séquence d'ADN simple brin. En effet, contrairement à l'ensemble des préjugés de l'homme de l'art, et à l'état de la technique limité à une étape classique d'amplification par PCR, c'est-à-dire sur une molécule d'ADN double brins, la présente invention décrit pour la première fois un procédé de génération d'une banque d'anticorps à partir d'une seule amorce primaire et de la séquence d'ADNc simple brin obtenue à partir de cette amorce primaire.

Selon un premier aspect, l'invention objet de la présente demande de brevet vise un procédé de génération d'une séquence d'ADN codant pour une chaîne lourde et/ou une chaîne légère d'au moins un anticorps à partir d'un extrait ou d'un mélange d'ARN issu d'une cellule, cette cellule étant capable d'exprimer de l'ARN codant pour un anticorps ou au moins pour la chaîne lourde et/ou la chaîne légère d'un anticorps, caractérisé en ce que ledit procédé comprend au moins les étapes suivantes :
a) la mise en contact d'une amorce primaire (I) avec ledit extrait ou mélange d'ARN susceptible de contenir au moins un ARN, dénommé ici ARN « sens » codant pour la chaîne lourde ou la chaîne légère d'un anticorps, cette amorce primaire étant capable de s'hybrider spécifiquement à un fragment de la séquence dudit ARN, « sens » compris dans la séquence codant pour la région constante C de la chaîne lourde et/ou légère dudit anticorps ;
b) synthétiser, à partir de ladite amorce primaire (1), un ADNc simple brin dénommé ADNc « anti-sens » ;
c) éliminer, le cas échéant, les amorces primaires (1) n'ayant pas hybridé à l'étape a) ;
d) circulariser ledit ADNc « anti-sens » en introduisant une liaison covalente entre son extrémité 5' et son extrémité 3' ;
e) la mise en contact d'une amorce secondaire (II), dénommée amorce secondaire « sens », avec ledit l'ADNc circulaire « anti-sens » obtenu à l'étape d), cette amorce secondaire « sens » (II) étant capable de s'hybrider audit ADNc circulaire « anti-sens » ;
f) amplifier ledit ADNc « anti-sens » à partir de ladite amorce secondaire (II) ; et
g) récupérer le brin d'ADN linéaire complémentaire « sens » ainsi amplifié.

Le recours à une étape de PCR nécessite de connaître les séquences respectivement des extrémités 5' et 3' du gène qui doit être amplifié afin de pouvoir y hybrider des amorces complémentaires et ainsi initier l'amplification par les techniques classiques des molécules double brins.

Le principe même de l'invention repose sur la capacité à amplifier une séquence d'ADN simple brin circularisée à l'aide d'une enzyme appropriée. Il apparaît donc clairement à l'homme de l'art que seule une amorce complémentaire de l'extrémité 3' de la séquence devant être amplifiée est nécessaire. Plus particulièrement, dans le cadre de la génération d'une séquence codant pour un anticorps, seule une amorce complémentaire de tout ou partie de la séquence correspondant à la région constante devient alors nécessaire pour l'amplification selon l'invention. Une fois l'amorce fixée sur la molécule d'ADN simple brin, dans le cas présent la molécule d'ARN « sens », il suffit d'amplifier cette dernière à l'aide d'une enzyme appropriée capable d'amplifier une séquence simple brin.

L'homme de l'art comprendra aisément l'avantage apporté par l'invention objet de l'invention en ce sens que cette dernière permet d'amplifier intégralement la séquence de l'ARN « sens » correspondant à la région variable, et ce jusqu'à son extrémité 5', c'est-à-dire incluant, le cas échéant, les séquences correspondant au peptide signal ainsi qu'à la région 5'-UTR.

Cet aspect est particulièrement avantageux, en ce sens que l'anticorps isolé par cette technique peut alors être cloné directement avec son peptide signal d'origine, ce qui est optimal pour l'expression de tels anticorps dans un système d'expression eucaryote. En effet, sans la présence du peptide signal d'origine, le clonage d'anticorps dans un vecteur d'expression oblige à ajouter un peptide signal non natif, ce qui nécessite toujours une connaissance de la séquence de l'extrémité 5' du gène de l'anticorps et peut introduire des modifications de séquences nucléiques et/ou protéiques de tels anticorps. En outre, un tel procédé permet de s'affranchir de l'ensemble des inconvénients décrits plus haut.

Il est bien évident que chaque étape décrite plus haut peut être remplacée par une étape équivalente permettant d'obtenir le résultat essentiel recherché pour cette étape sans pour autant sortir de l'objet de la présente invention et de l'étendue de la protection souhaitée pour ce procédé. Par soucis de clarté, il est ici précisé que, dans l'ensemble de la présente demande de brevet, les expressions « sens » et « anti-sens » ont pour référentiel, de manière classique pour l'homme de l'art, le sens 5'3' de la molécule d'ARN initiale, c'est-à-dire de la molécule d'ARN codant pour la chaîne lourde ou la chaîne légère d'un anticorps.

En conséquence, et logiquement, l'amorce primaire (I) et la molécule d'ADNc générée à partir de cette amorce, toutes deux étant complémentaires dudit ARN, seront donc caractérisées par l'expression « anti-sens ». De la même manière, l'amorce secondaire (II) ainsi que la molécule d'ADN linéaire générée à partir de cette amorce, toutes deux complémentaires de l'ADNc généré et de ce fait orientées comme la séquence d'ARN initiale, seront caractérisées par l'expression « sens ». Enfin, de la même manière, l'amorce tertiaire (III) et la séquence d'ADN complémentaire générée à partir de cette amorce tertiaire seront, quant à elles, caractérisées par l'expression « anti-sens ».

Selon une forme d'exécution préférée de l'invention, les cellules dont sont issus les extraits ou mélanges d'ARN sont des cellules de mammifères, de préférence de souris, de rat ou de primates, l'origine humaine étant la plus préférée.

Dans un mode de réalisation préféré, l'extrait ou le mélange d'ARN est un extrait ou mélange d'ARN totaux, un extrait ou mélange d'ARN enrichi en ARN messager et/ou pré-messager.

Selon un second mode de réalisation, le procédé objet de l'invention peut être appliqué directement à des cellules naturelles, saines ou issues d'un tissu ou organe pathologique, notamment d'une tumeur, ces cellules étant capables d'exprimer des ARN codant pour une chaîne lourde ou légère d'un anticorps. Le procédé objet de l'invention peut être appliqué également à des cellules d'une lignée cellulaire issue de ces cellules saines ou pathologiques.

Selon une forme d'exécution également préférée de l'invention, les extraits ou mélanges d'ARN sont issus de splénocytes, de nodules ou de lymphocytes B.

De manière préférée, les cellules dont sont issus les extraits ou mélanges d'ARN sont des lymphocytes B ou l'un de ses progéniteurs, ou un plasmocyte. De préférence, ces cellules sont choisies parmi les cellules progéniteur pro-B (cellules souches déjà engagées dans le linéage B), les cellules précurseurs pré-B, les lymphocytes B immatures, les lymphocytes B matures immunocompétents ou les plasmocytes (voir notamment pour les caractéristiques de ces cellules, les définitions données dans la publication « Différenciation des lymphocytes B » par les Professeurs Marie-Paule LEFRANC et Gérard LEFRANC, Université Montpellier II et Laboratoire d'ImmunoGénétique Moléculaire, LIGM, sur le site d'ImmunoGenetics « http://imgt.cines.fr/textes/IMGTeducation/Tutorials/IGandBcells/_FR/Differenciation B/ »).

Selon une forme d'exécution très préférée de l'invention, les extraits ou mélanges d'ARN sont issus de lymphocytes B.

Selon un mode de réalisation particulier, les cellules dont sont issus les ARN sont des cellules de lignée cellulaire, des cellules isolées de mammifère sain ou préalablement traité avec un principe actif thérapeutique, notamment un antigène à titre d'immunogène ou comme vaccin, ce dernier pouvant être un vaccin anti-infectieux ou anti-tumoral. Parmi ces cellules, on peut également citer des cellules issues d'un patient atteint d'un cancer ou encore infecté par un virus pathogène.

Ainsi, un avantage de l'invention est de permettre de travailler à partir d'une grande variété de matière cellulaire.

Par exemple, selon au autre mode de réalisation de l'invention, il peut être souhaité de travailler à partir de cellules transformées comme, par exemple, des hybridomes résultant d'une fusion entre d'une part des lymphocytes B de la rate et d'autre part des cellules de myélome.

Dans cette application, un des intérêts du procédé selon l'invention peut être de permettre l'obtention de la séquence native de l'anticorps monoclonal exprimé par ledit hybridome sans amplification par PCR et donc sans connaissance de la séquence de l'extrémité 5' dudit anticorps. La séquence obtenue comprend également la séquence codant pour le peptide signal natif dudit anticorps et pour la région 5' UTR. Sur cette base, l'anticorps peut être cloné par les moyens classiques connus de l'homme de l'art ou bien, selon une autre forme de réalisation, le gène correspondant audit anticorps peut être synthétisé en entier sans nécessiter d'étape de PCR.

Ainsi, dans un mode de réalisation particulier du procédé selon l'invention, ladite cellule capable d'exprimer un anticorps est en un hybridome.

Dans un mode de réalisation particulièrement préféré, la synthèse de l'ADNc à l'étape b) à partir d'un ARN et d'une amorce primaire (I) est réalisée par toute technique bien connue de l'homme de l'art pour obtenir la synthèse d'un ADNc à partir d'une amorce, notamment en présence d'une transcriptase inverse et d'un mélange de nucléotides (dNTP).

L'amorce primaire (I) utilisée pour la synthèse dudit ADNc comprend au niveau de son extrémité 5' un groupement capable de lier par liaison covalente à l'extrémité 3' de l'ADNc synthétisé à l'étape b), et ainsi aboutir à la circularisation de cet ADNc, ce groupement pouvant être introduit lors de la synthèse de l'amorce primaire (I). Parmi ces groupements, on préfère le groupement PO₄ ce groupement pouvant former une liaison covalente de type phosphodiester avec l'extrémité 3' OH dudit ADNc synthétisé en présence d'une ADN ligase.

Dans un mode de réalisation particulier, préalablement à l'étape d) de circularisation, il est préféré de s'assurer qu'il ne reste aucun reliquat d'amorce primaire (I) non hybridée dans le milieu de réaction, ceci afin d'éviter toute circularisation de ladite amorce (I) entraînant alors l'amplification de ladite amorce (1) circularisée plutôt que de l'ADNc circularisé de l'anticorps.

Pour ce faire, plusieurs solutions sont envisageables par l'homme de l'art comme, à titre d'exemple non limitatif, un lavage du milieu de réaction suite à la synthèse d'ADNc par filtration par taille, ou avec les produits de purification des ADNc sachant que ces derniers excluent tout matériel en dessous de 100 paires de base (pb), ou encore par purification par séparation sur gel d'agarose. Selon une autre alternative, les quantités d'amorce primaire (I) utilisées seront optimisées de manière à être en quantité sous saturante, l'étape c) n'étant pas nécessaire dans cette configuration.

Selon une forme d'exécution préférée de l'invention, ladite amorce primaire (I) est spécifique d'une séquence située à, ou adjacente à, l'extrémité 5' de la séquence d'ARN « sens » correspondant à la région constante C.

Cet aspect de l'invention est avantageux en ce sens qu'il permet de synthétiser puis d'amplifier spécifiquement l'ADNc circularisé d'un anticorps à partir d'une amorce reconnaissant spécifiquement au moins une partie de la séquence codant pour la région constante d'une chaîne lourde ou d'une chaîne légère d'un anticorps.

Dans un mode de réalisation particulièrement préféré, l'amorce primaire (I) utilisée pour la synthèse dudit ADNc comprend au niveau de son extrémité 5' un groupement capable de lier par liaison covalente à l'extrémité 3' de l'ADNc synthétisé à l'étape b), et ainsi aboutir à la circularisation de cet ADNc, ce groupement pouvant être introduit lors de la synthèse de l'amorce primaire (I). Parmi ces groupements, on préfère le groupement PO₄. ce groupement pouvant former une liaison covalente de type phosphodiester avec l'extrémité 3' OH dudit ADNc synthétisé en présence d'une ADN ligase.

La séquence d'ADNc étant circularisée, l'homme de l'art comprendra que l'amorce secondaire (II) peut être spécifique ou non, cette dernière amplifiant en « cercles roulants », l'intégralité de la séquence de l'ADNc sera amplifiée. Selon un mode d'exécution de l'invention, il peut être utilisé des amorces aléatoires.

Selon une forme d'exécution préférée de l'invention, ladite amorce secondaire (II) est spécifique de la séquence comprise entre l'extrémité 5' de ladite séquence d'ADNc « anti-sens » et l'extrémité 3' de la séquence dudit ADNc « anti-sens » correspondant à la région constante C.

De manière encore plus préférée, ladite amorce secondaire (II) est spécifique d'une séquence située à, ou adjacente à, l'extrémité 5' de la séquence d'ADNc « anti-sens ».

Comme il ressortira clairement des figures, l'extrémité 5' de la séquence d'ADNc "anti-sens" correspond également à l'extrémité 5' de l'amorce primaire (I), la séquence dudit ADNc "anti-sens" étant bien évidemment initiée par l'amorce primaire (I).

Selon une forme d'exécution possible de l'invention, ladite amorce secondaire (II) mise en oeuvre à l'étape e) du procédé de l'invention, consiste en une amorce « anti-sens » capable de s'hybrider avec l'ensemble de la séquence codant pour l'amorce primaire (I). Selon une autre forme d'exécution possible, l'amorce secondaire (II) est de taille inférieure à la taille de l'amorce primaire (1) et vient s'hybrider au sein de la séquence codant pour l'amorce primaire (I).

Plus particulièrement, la présente invention vise un procédé caractérisé en ce que lesdites amorces primaire (I) et secondaire (II) consistent chacune en une séquence d'ADN simple brin ayant une longueur comprise entre 10 et 100 nucléotides.

De manière encore plus préférée, le procédé selon l'invention est caractérisé en ce que lesdites amorces primaire (I) et secondaire (II) consistent respectivement chacune en une séquence d'ADN simple brin ayant une longueur comprise entre 20 et 60 nucléotides pour l'amorce primaire (I) et entre 10 et 30 nucléotides pour l'amorce secondaire (II).

De manière classique, la synthèse du brin d'ADNc « anti-sens » est réalisée par addition de nucléotides libres et d'une enzyme de type transcriptase reverse comme, à titre d'exemple non limitatif, la transcriptase reverse Superscript III, ou encore l'Avian myeloblastosis virus ou Moloney Murine Leukaemia Virus reverse transcriptase.

De manière préférée, le procédé selon l'invention comprend, préalablement à l'étape a) d'hybridation de l'amorce primaire (I), une étape de dénaturation de l'ARN.

La dénaturation de l'ARN récupéré peut être réalisée classiquement par toute méthode connue de l'homme de l'art. A titre d'exemple non limitatif, peut être mentionné le chauffage à 65°C, ou plus, du mélange réactionnel, ce dernier permettant de dénaturer toute structure secondaire de l'ARN. La présence de l'amorce primaire (I) dans le mélange réactionnel permet, lors du refroidissement post-dénaturation, l'hybridation de l'amorce primaire à sa séquence complémentaire dans ledit ARN avec une forte spécificité. En complément, peu être ajouté, par exemple, un cofacteur protéique comme le cofacteur T4 gène 32. Cela permet, entre autre, de réduire la synthèse non spécifique d'ADNc.

Une des étapes clé du procédé selon l'invention consiste en la circularisation de l'ADNc « anti-sens ». Cette étape peut être réalisée par toute technique connue de l'homme de l'art. En effet, la possibilité de s'affranchir des étapes classiques de PCR pour l'amplification repose en grande partie sur le fait que la molécule d'ADNc qui sera amplifiée, sous une forme simple brin, est circularisée. Si le principe de circularisation de l'ADN n'est pas nouveau en tant que tel, son application dans le cadre de la présente invention, à savoir la génération d'une séquence codant pour une chaîne lourde et/ou légère d'un anticorps est nouvelle et n'a jamais été décrite à ce jour.

Cette étape de circularisation peut être réalisée par toute technique connue de l'homme de l'art dès lors qu'elle permet de lier par liaison covalente les extrémités 5' et 3' d'un même ADN monocaténaire (simple brin).

De manière préférée, ladite étape de circularisation est réalisée par mise en contact avec une ligase de type « ssDNA ligase » (« ssDNA » pour « single strand DNA » ou ADN simple brin).

Selon une forme de réalisation, la ligase est sélectionnée parmi la CircLigase™ (Epicentre, Madison, WI, USA) ou la thermophage ssDNA ou toute enzyme équivalente.

Toutefois, selon une forme de réalisation préférée du procédé selon l'invention, la ligase consiste en la CircLigase™ ou toute enzyme équivalente.

Pour plus d'élément, l'homme de l'art peut se reporter à la publication suivante : (Polidoros, A.N. et al., Biotechniques 41(1), 35 (2006)).

De manière préférée, la ligase utilisée introduit une liaison covalente entre l'extrémité 3' OH et l'extrémité 5' PO₄ de l'ADNc. Pour ce faire, un groupement PO₄ a été préalablement introduit au niveau de l'extrémité 5' lors de la synthèse de l'amorce primaire (I) utilisée pour la synthèse dudit ADNc. Bien évidemment, tout autre groupement pouvant assurer la même fonction, c'est-à-dire permettre la liaison covalente entre les extrémités 5' et 3' de la molécule d'ADNc peut également être utilisé.

Pour faciliter le clonage d'un anticorps isolé par cette approche, l'amorce primaire (1) peut comprendre un site de restriction natif au sein de la séquence de la partie constante, ou voir même une séquence non-native spécifiquement ajoutée pour faciliter cette étape. Cette séquence ajoutée comme adaptateur peut faciliter le clonage dans un vecteur d'expression. L'adaptateur peut comprendre, de préférence, un ou plusieurs site(s) de restriction compatible(s) avec des sites de restriction présents dans le vecteur d'expression. Un deuxième exemple peut consister en un adaptateur comprenant deux séquences d'ADN reconnues par une enzyme type site spécifique recombinase avec un site de restriction entre eux pour générer des monomères. Ledit site spécifique recombinase à titre d'exemple non limitatif, peut être le lambda phage site spécifique recombinase ou bactériophage PI recombinase Cre. Cet adaptateur peut aussi comprendre une séquence d'ADN comprenant un site de restriction pour la préparation du monomère avec les séquences de chaque côté adaptées aux « ligase independant cloning ».

Une autre étape clé du procédé objet de l'invention réside dans l'étape d'amplification à proprement parler de la molécule d'ADNc « anti-sens » circulaire. Comme mentionné plus haut, cette étape est initiée par l'hybridation, dans un premier temps, d'une amorce secondaire (II) entre l'extrémité 5' de la séquence dudit ADNc « anti-sens » et l'extrémité 3' de la séquence correspondant à la région constante C dudit même ADNc « anti-sens ».

Cette amorce secondaire est choisie selon les manières connues par l'homme de l'art sachant qu'une complémentarité avec la séquence d'ADNc codant pour la partie constante d'un ou plusieurs isotype(s) d'immunoglobulines et comprise entre l'extrémité 5' de ladite partie constante et l'extrémité hybridée par l'amorce primaire (1) est souhaitée.

Afin d'amplifier la molécule d'ADNc, l'étape f) d'amplification est mise en oeuvre à l'aide d'une enzyme capable d'amplifier une séquence d'ADN circulaire simple-brin.

Plus particulièrement, ladite enzyme consiste préférentiellement en la BSE DNA polymérase large fragment (BSE ADN polymérase, grand fragment), la « rolling circle polymerase » (polymérase pour « cercle roulant ») du bactériophage φ29 ou toute enzyme équivalente capable d'amplifier une séquence d'ADN circulaire simple-brin. Dans une première application, il peut être envisagé de séquencer directement l'ADN simple brin amplifié consistant en plusieurs copies de l'anticorps par les approches classiques et connues de l'homme de l'art. La séquence obtenue par cette approche comprend les séquences codant pour la région 5'-UTR, le peptide signal et la partie variable dudit anticorps sans qu'il ait été nécessaire de connaître préalablement l'extrémité 5' de la séquence codant pour l'anticorps.

Selon une forme de réalisation, le procédé selon l'invention comprend, en outre, une étape de séquençage du brin d'ADN linéaire « sens » récupéré à l'étape g).

Cet aspect présente un intérêt, par exemple et notamment, lorsque l'ADNc « anti-sens » a été obtenu à partir de l'ARN récupéré d'un hybridome.

Dans une seconde application, le procédé objet de l'invention peut être mis en oeuvre dans un procédé pour générer une banque d'ADN codant pour des chaînes lourdes ou légères d'anticorps, préférentiellement des anticorps de mammifères tels que la souris, le rat ou de primates, l'origine humaine étant préférée.

Plus particulièrement, dans ce cas, l'invention a pour objet un procédé de génération d'une banque d'ADN codant pour des chaînes lourdes ou légères d'anticorps, ledit procédé étant caractérisé en ce qu'il consiste à mettre en oeuvre le procédé tel que décrit plus haut et en ce qu'il comprend, en outre, les étapes suivantes :
h) la mise en contact d'une amorce tertiaire (III), dénommée amorce tertiaire «anti- sens », avec ledit ADN linéaire «sens » obtenu à l'étape g), cette amorce tertiaire « anti-sens » (III) étant capable de s'hybrider spécifiquement à un fragment de la séquence de l'ADN linéaire comprise entre l'extrémité et l'extrémité 3' et 5' dudit brin d'ADN linéaire et correspondant à la séquence de cet ADN linéaire codant pour la région constante C ;
i) générer, à partir de ladite amorce tertiaire (III), un concatémère par synthèse du brin d'ADN « anti-sens » complémentaire, et
j) cloner ledit concatémères d'ADN double brin ainsi obtenu au sein de vecteurs préalablement préparés.

Un tel procédé permet par exemple de séquencer les chaînes lourdes ou légères représentées par les monomères de ces concatémères à partir de la librairie de concatémères ainsi obtenue et clonée.

Pour un tel procédé compris dans l'objet de la présente invention, une étape de séquençage du concatémère ou d'au moins un monomère dudit concatémère sera mise à oeuvre à la fin de l'étape j).

Par l'expression « concatémère », il faut comprendre une molécule d'ADN constituée d'un même monomère répété et formant un multimère linéaire.

Plus particulièrement, selon une forme d'exécution préférée, ladite amorce tertiaire (III) est spécifique d'une séquence située à, ou adjacente à, l'extrémité 5' de la séquence de l'ADN linéaire « sens » correspondant à la région constante C.

Cet aspect présente l'avantage de pouvoir synthétiser un double brin complémentaire au concatémère simple brin et d'initier ensuite une amplification arborescente.

Comme pour les amorces primaires et secondaires, ladite amorce tertiaire (III) consiste en une séquence d'ADN simple brin ayant une longueur comprise entre 10 et 100 nucléotides. Une longueur comprise entre 15 et 60 nucléotides, 15 et 50, 15 et 40, 15 et 35, et 15 et 30 pour ladite amorce tertiaire (III) est encore plus préférée.

Dans un mode de réalisation préféré, l'invention a pour objet un procédé pour générer une banque d'ADN codant pour des chaînes lourdes ou légères d'anticorps selon l'invention, caractérisé en ce que ce procédé comprend une étape préalable à l'étape j) de clonage, dans laquelle ledit concatémère obtenu est segmenté au niveau des séquences correspondant aux amorces utilisées, cette étape étant suivie de l'étape j) de clonage des segments d'ADN double brins ainsi obtenus au sein de vecteurs préalablement préparés.

Dans ce procédé pour générer une banque d'ADN codant pour des monomères de chaînes lourdes ou légères d'anticorps, il est préféré que l'amorce primaire (I) comprend un site de restriction et qu'ainsi ledit concatémère obtenu est segmenté au niveau de la séquence de cette amorce primaire.

La présence de ce site de restriction est un des moyens qui peut être utilisé pour segmenter le concatémère préalablement à l'étape j). De par la nature même de la génération du brin complémentaire constituant le concatémère double brin, le site de restriction se retrouve sur les deux brins formant ledit concatémère. Selon une première forme de réalisation, l'amorce primaire (I) peut être sélectionnée pour comprendre naturellement au moins un site de restriction. A titre d'exemple, on peut citer *Hpa* I et/ou *Hinc* II présent dans la partie constante des chaînes légères κ ou encore *Bgl* II pour les chaînes λ.

Selon une deuxième forme de réalisation, l'amorce primaire (I) peut être légèrement modifiée de manière à comprendre un site de restriction. Par exemple, il est possible de muter un ou deux résidus afin de créer artificiellement un site de restriction sans pour autant faire perdre à ladite amorce sa capacité à s'hybrider spécifiquement avec le domaine de l'ARN sens souhaité.

Enfin, selon une troisième forme de réalisation, la séquence codant pour un site de restriction peut être insérée au sein de l'amorce primaire (I), ceci en conservant pour ladite amorce sa capacité à s'hybrider spécifiquement avec le domaine de l'ARN sens souhaité.

Le procédé pour générer une banque d'ADN codant pour des segments monomériques de chaînes lourdes ou légères d'anticorps selon l'invention est donc aussi caractérisé en ce que ladite étape de segmentation du concatémère est mise en oeuvre par digestion enzymatique à l'aide d'endonucléase de restriction spécifiques du site de restriction compris ou introduit dans l'amorce primaire (I).

Ledit monomère peut ensuite être cloné dans un vecteur d'expression eucaryote comprenant la suite de la partie constante 3' de ladite amorce primaire (I) de la chaîne lourde ou légère. Le clonage peut s'effectuer via un site de restriction complémentaire à celui présent au sein de l'amorce primaire (I) ou par recombinaison site spécifique. Ledit vecteur d'expression doit comprendre les éléments nécessaires pour l'expression d'une protéine recombinante dans une souche cellulaire hôte. A titre d'exemple non limitatif, le vecteur pCEP (Invitrogen) comprend un promoteur d'expression approprié.

Plus particulièrement, le procédé selon l'invention est caractérisé en ce que le vecteur utilisé pour l'étape j) comprend en outre les séquences codant pour les domaines constants de la chaîne lourde ou légère d'une immunoglobuline.

De manière préférée, ladite séquence codant pour le domaine constant de la chaîne lourde consiste préférentiellement en une séquence issue d'une immunoglobuline avec ancrage membranaire, ou comprenant une région C-terminale transmembranaire.

Pour faciliter le criblage d'une banque d'anticorps générée selon l'invention, lesdites séquences de chaînes lourdes ou légères d'anticorps peuvent être clonées, par exemple, au niveau de l'extrémité 5' de la séquence signale de liaison glycosidylphosphotidylinositol d'une région transmembranaire issue du facteur accélérateur dissociation humain (« decay-accelerating factor », « DAF » ou CD55). Ledit vecteur ainsi construit doit être ensuite transfecté dans une souche eucaryote hôte comme, par exemple, les cellules CHO, COS, HEK, NIH-3T3.

En conséquence, l'invention décrit un procédé de criblage d'une banque de cellules capables d'exprimer une librairie d'ADN codant pour des monomères de chaînes lourdes ou légères d'anticorps, ladite librairie ayant été générée par le procédé selon l'invention, caractérisée en ce qu'il comprend, en outre, une étape k) consistant à transfecter avec le vecteur obtenu à l'étape j) du procédé de génération de la banque d'ADN selon l'invention, une cellule hôte capable d'exprimer l'anticorps codé par le fragment d'ADN double brin inséré au sein dudit vecteur, et une étape 1) dans laquelle les cellules exprimant de tels ADN codant pour des séquences de chaînes lourdes ou légères d'anticorps sont sélectionnées.

Plus particulièrement, ladite cellule hôte de l'étape 1) consiste en une cellule capable d'exprimer à sa surface la chaîne lourde et/ou la chaîne légère de l'anticorps codé par le fragment d'ADN double brin inséré, et encore plus préférentiellement, ladite cellule hôte consiste en une cellule eucaryote, comme, par exemple, les cellules CHO, COS, HEK, NIH-3T3.

L'expression dans une cellule hôte eucaryote présente l'avantage d'exprimer la forme native de l'anticorps. Les transfections de plusieurs cellules avec un mélange d'anticorps clonés permet de générer une banque d'anticorps (ou fragments comprenant la chaîne lourde et/ou légère) liés ou exprimés à la surface de la cellule hôte. Lesdites banques d'anticorps (ou fragments) peuvent être ensuite criblées par toute approche connue de l'homme de l'art comme, par exemple, par séparation des clones positifs par FACS ou l'isolation de populations polyclonales avec une cible immobilisée ou en suspension.

Selon un autre aspect, il est envisagé l'utilisation d'un procédé selon l'invention pour la génération d'une librairie d'anticorps ou leurs fragments comprenant la chaîne lourde et/ou légère.

De manière similaire, l'invention couvre également toute librairie d'anticorps obtenue par mise en oeuvre du procédé tel que décrit plus haut.

De manière préférée, mais sans aucune limitation, la librairie d'anticorps selon l'invention est une librairie d'anticorps, ou leurs fragments, humains.

Selon encore un autre aspect de l'invention il est décrit et revendiqué tout anticorps, fragment fonctionnel ou composé dérivé, obtenu par criblage d'une librairie obtenue selon l'invention, lesdits anticorps ou fragments consistant préférentiellement en des anticorps humains.

Enfin, selon un dernier aspect de l'invention, il est envisagé un procédé *in vitro* de screening d'anticorps, ou de leurs fragments fonctionnels capables de reconnaître l'épitope spécifique reconnu par l'anticorps dont il est issu, ces anticorps étant actifs contre une maladie donnée, ce procédé comprenant une étape dans laquelle on récupère un échantillon de sang d'un patient souffrant de ladite maladie donnée et on applique le procédé selon l'invention audit échantillon de sang. De manière encore plus préférée, bien que non limitative, ladite maladie donnée est un cancer.

D'autres caractéristiques et avantages de l'invention apparaissent dans la suite de la description avec les exemples et les figures dont les légendes sont représentées ci-après.

### LEGENDE DES FIGURES

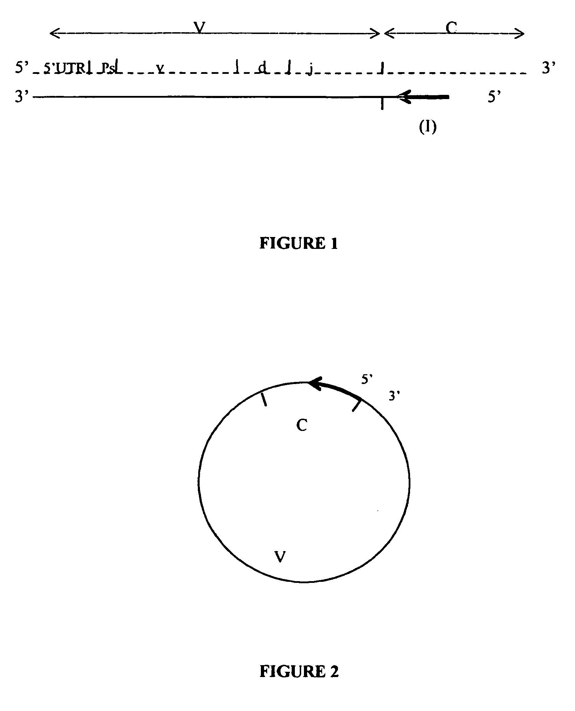

La figure 1 est un schéma illustrant les étapes a) et b) d'un mode de réalisation du procédé objet de l'invention.
La figure 2 est un schéma illustrant l'étape c) d'un mode de réalisation du procédé objet de l'invention.

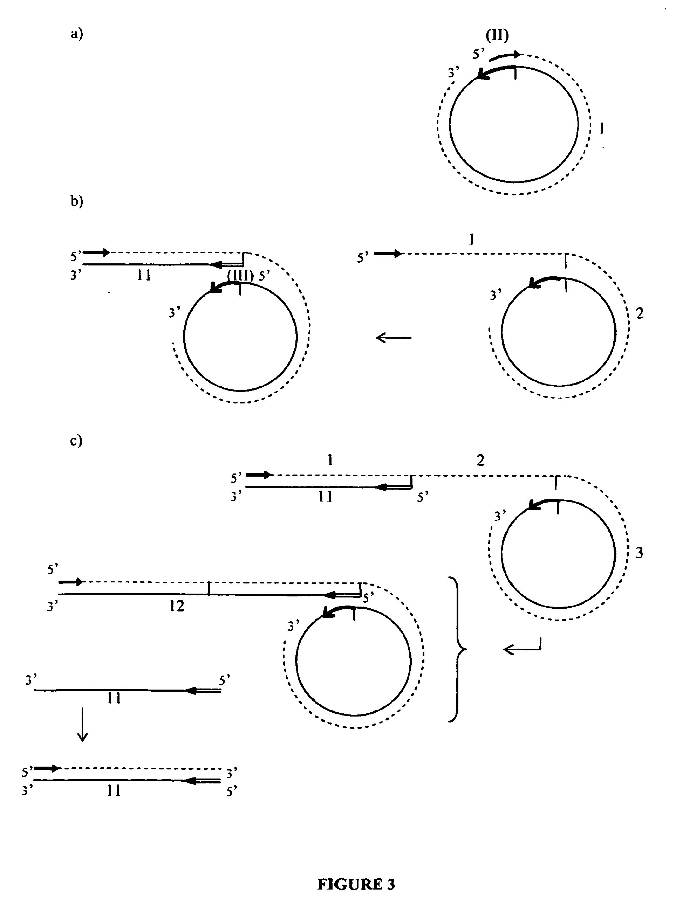

La figure 3 est un schéma illustrant les étapes d), e) et f) d'un mode de réalisation du procédé objet de l'invention.

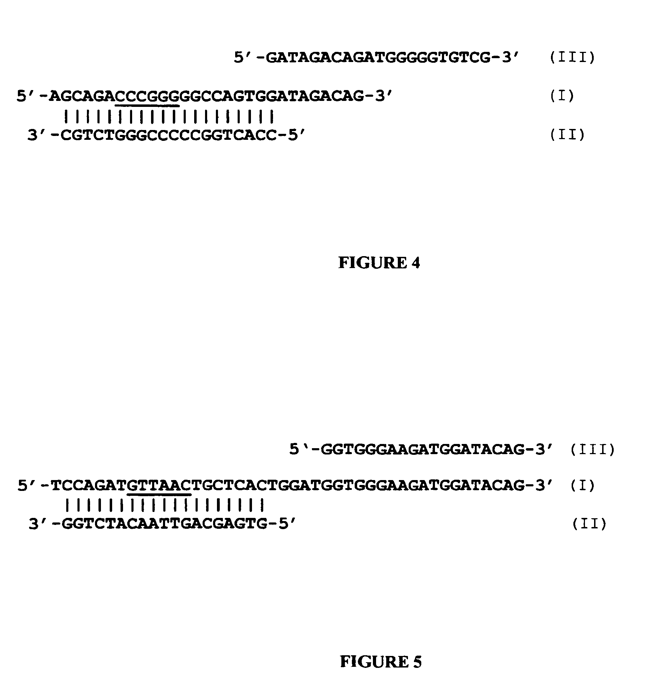

La figure 4 représente les différentes amorces primaire (I), secondaire (II) et tertiaire (III) utilisées pour la génération de la séquence de la chaîne lourde dans l'exemple 2. Le site de restriction se trouve souligné au niveau de l'amorce primaire (I).
La figure 5 représente les différentes amorces primaire (I), secondaire (II) et tertiaire (III) utilisées pour la génération de la séquence de la chaîne légère dans l'exemple 2. Le site de restriction se trouve souligné au niveau de l'amorce primaire (1).

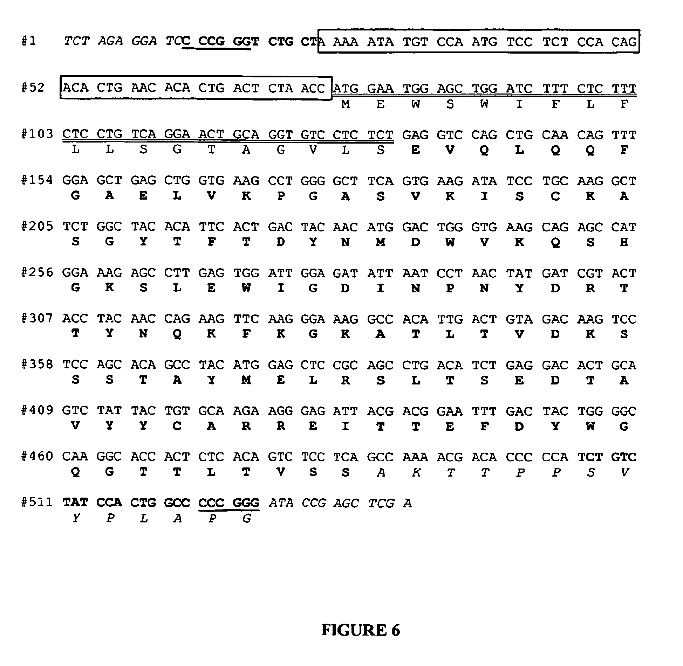

La figure 6 représente la séquence obtenue pour la chaîne lourde totale après séquençage (correspondant à la SEQ ID N° 11) et dans laquelle se retrouve une portion du vecteur de clonage (séquence nucléique italique), le site de restriction (séquence nucléique soulignée), le fragment correspondant à l'amorce primaire I (séquence nucléique en gras), la région 5'-UTR (séquence nucléique encadrée), le peptide signal (séquence nucléique double soulignée), la partie variable de la chaîne lourde (séquence d'acide aminé en gras) et une portion de la partie constante (séquence d'acide aminé italique).

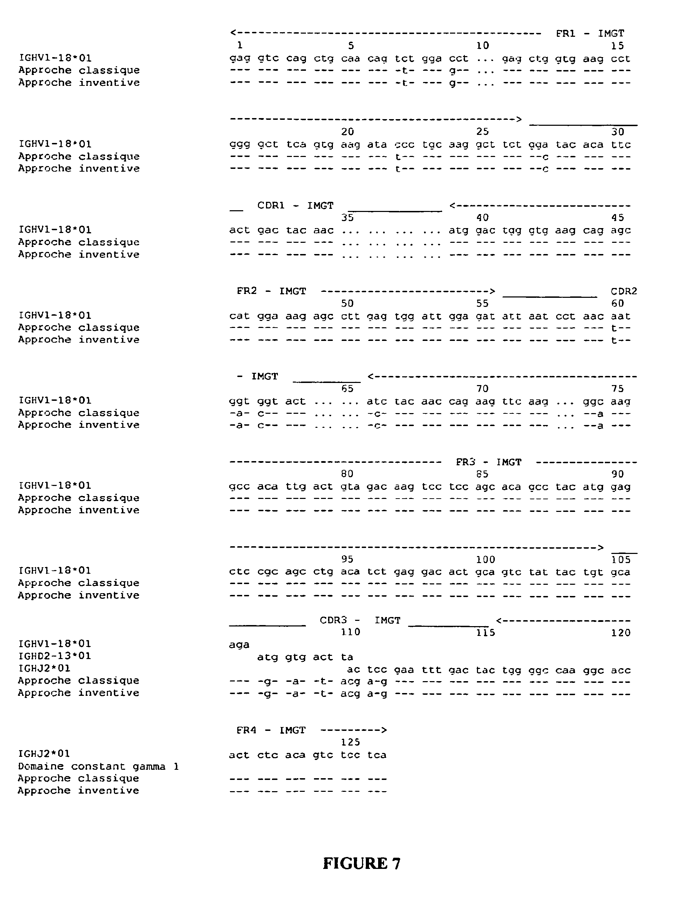

La figure 7 représente, pour la chaîne lourde, les alignements respectivement i) de la séquence de la lignée germinale IGHV1-18*01 dans la base IMGT, ii) de la séquence du domaine variable obtenue par approche classique et iii) de la séquence du domaine variable obtenue par le procédé objet de l'invention, appelée approche inventive sur la figure.

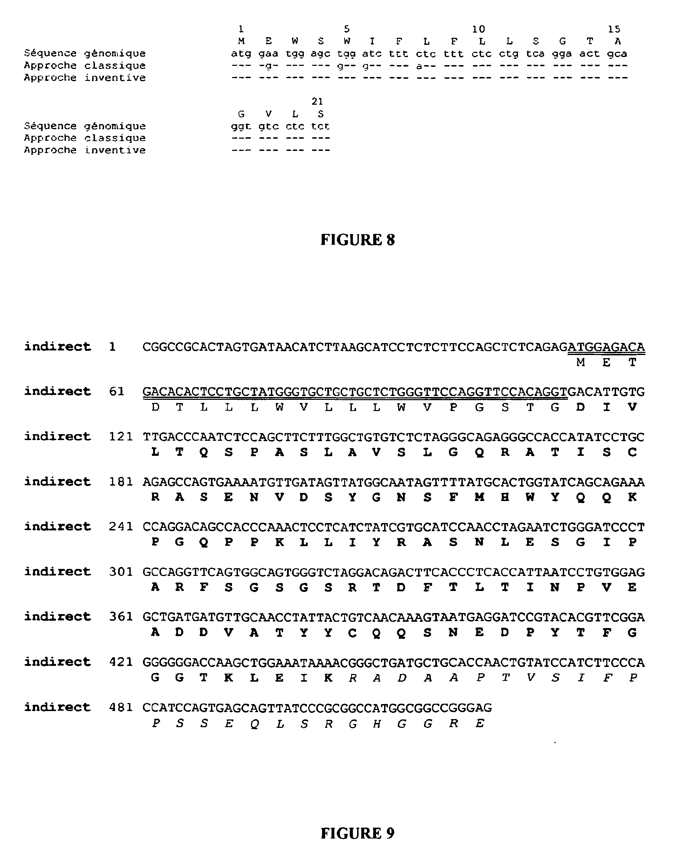

La figure 8 représente, toujours pour la chaîne lourde, les alignements respectivement i) de la séquence du peptide signal associé à la lignée germinale IGHV1-18*01 dans la base IMGT et appelée séquence génomique sur la figure, ii) de la séquence du peptide signal obtenue par approche classique, et iii) de la séquence du peptide signal obtenue par le procédé objet de l'invention, appelée approche inventive sur la figure.
La figure 9 représente la séquence nucléique et la séquence protéique obtenues pour la chaîne légère totale après séquençage (correspondant à la SEQ ID N° 12) dans laquelle la séquence nucléique doublement soulignée correspond au peptide signal, la séquence protéique en gras correspond au domaine variable, et la séquence protéique en italique correspond au domaine constant.

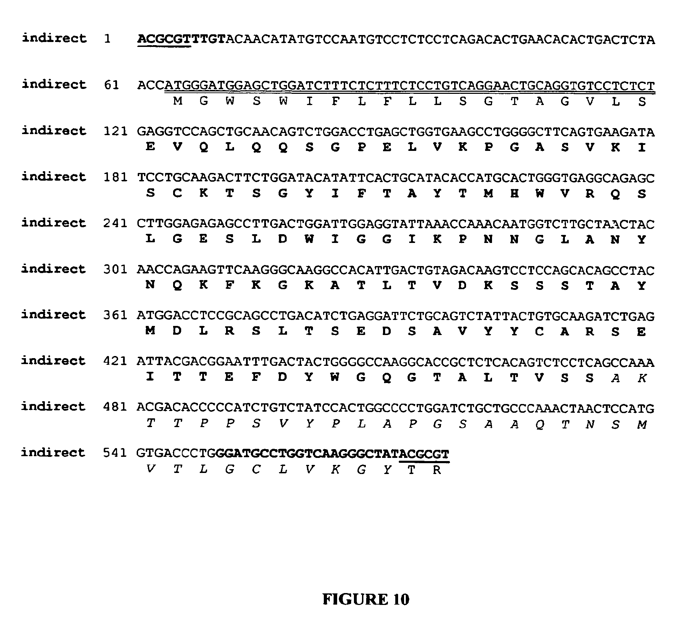

La figure 10 représente la séquence obtenue pour la chaîne lourde totale après séquençage indirect (correspondant à la SEQ ID N° 18) dans laquelle la séquence nucléique en gras correspond à l'amorce, la séquence nucléique soulignée correspond au site de restriction *Mlu* I, la séquence nucléique doublement soulignée correspond au peptide signal, la séquence protéique en gras correspond au domaine variable, et la séquence protéique en italique correspond au domaine constant.

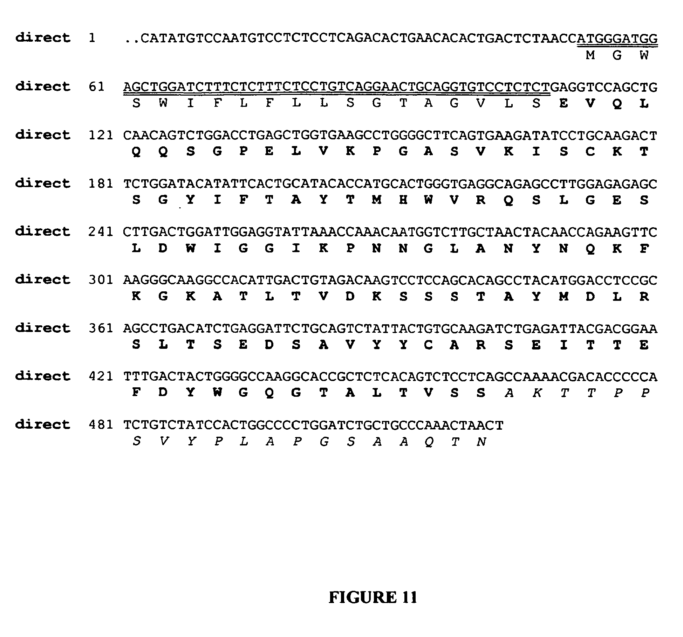

La figure 11 représente la séquence obtenue pour la chaîne lourde totale après séquençage combiné (correspondant à la SEQ ID N° 20) dans laquelle la séquence nucléique doublement soulignée correspond au peptide signal, la séquence protéique en gras correspond au domaine variable, et la séquence protéique en italique correspond au domaine constant.

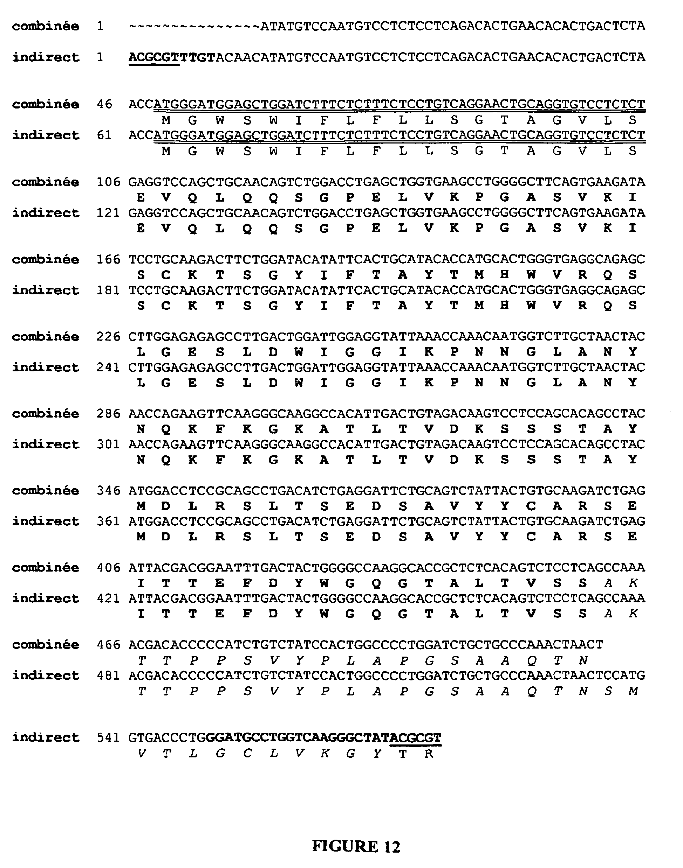

La figure 12 représente l'alignement des séquences obtenues pour la chaîne lourde totale après séquençage indirect (SEQ ID No. 18) et séquençage combinée (SEQ ID No. 20) dans lesquelles les séquences nucléiques en gras correspondent à l'amorce primaire, les séquences nucléiques soulignées correspondent aux sites de restriction *Mlu* I, les séquences doublement soulignées correspondent aux peptides signaux, les séquences protéiques en gras correspondent aux domaines variables, et les séquences protéiques en italique correspondent aux domaines constants.

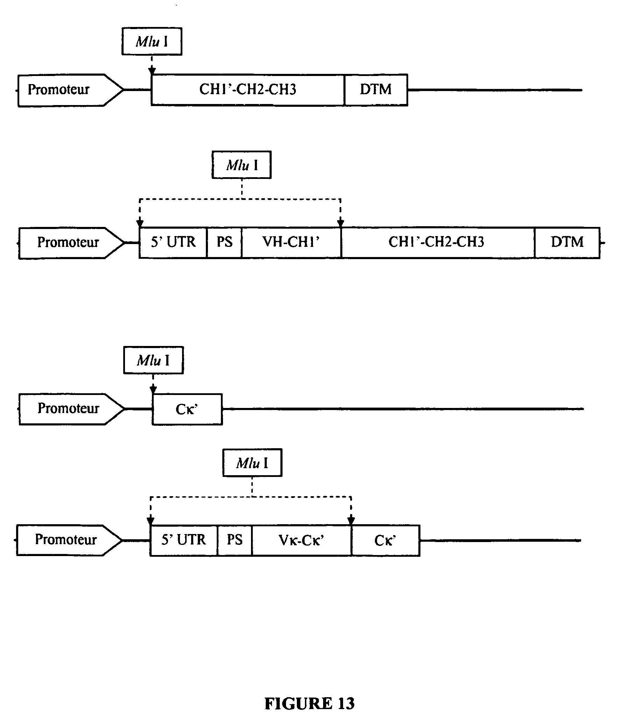

La figure 13 illustre un mode de clonage des parties variables des anticorps pour leur présentation à la surface des cellules eucaryotes. Promoteur: promoteur d'expression ie CMV; *Mlu* I : site de restriction spécifique pour le clonage des parties variables obtenues par RCA selon l'invention; CH1'-CH2-CH3 : domaine CH1 partiel et CH2-CH3 entier de la partie Fc d'une IgG1 humaine; DTM : domaine transmembranaire ; Cκ' : domaine Cκ2 humain partiel.

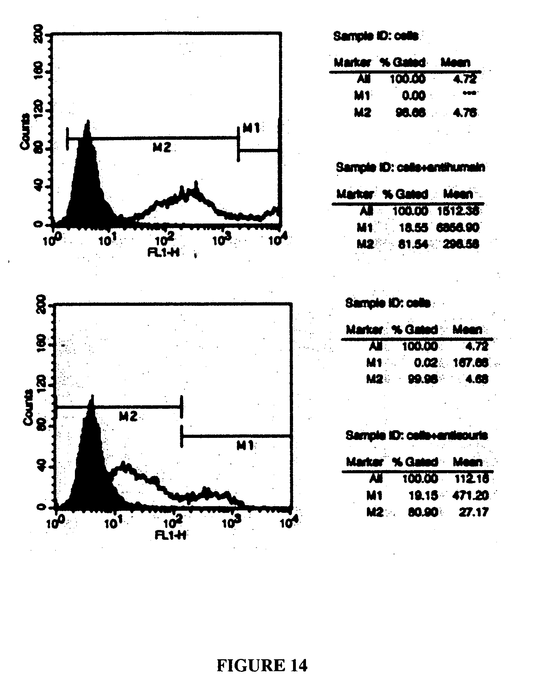

La figure 14 représente l'analyse par FACS de la présence d'anticorps à la surface des cellules CHO. Figure du haut : détection des anticorps à la surface avec anti-humain IgG Alexa 488. Figure du bas : détection des anticorps à la surface avec anti-murin IgG Alexa 488. Les cellules non-expresseur sont représentées par le pic noir, les cellules marquées par le trait noir.

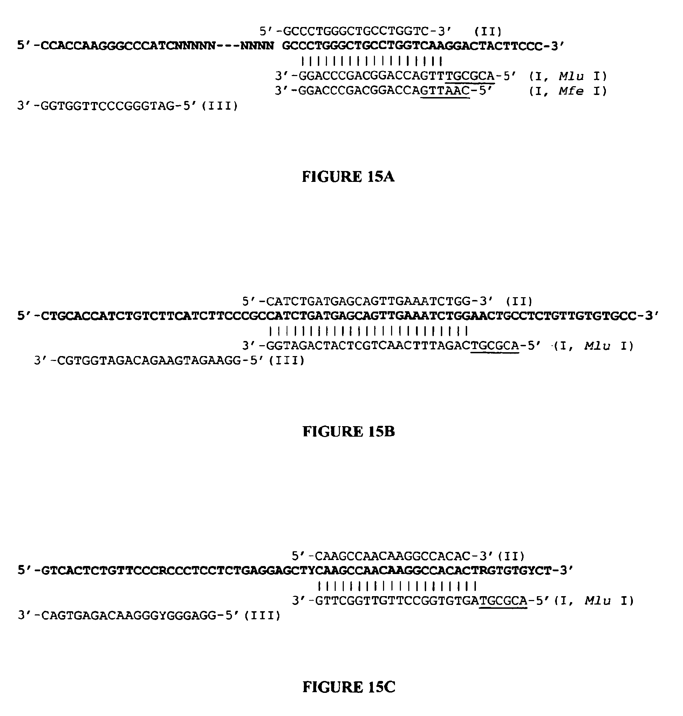

Les figures 15A, 15B et 15C représentent des exemples d'amorces avec :
   Figure 15A : Site des amorces pour le clonage et le séquençage des anticorps humains. Séquence consensus des partie constant humain IgG1-4 avec l'addition des sites de restriction *Mlu* I (totalement synthétique) ou *Mfe* I (modification une seul acide aminé) ;
   Figure 15B : Site des amorces pour le clonage et le séquençage des anticorps humains. Séquence consensus des parties constantes humaines Igκ avec l'addition du site de restriction *Mlu* 1 ; et
   Figure 15C : Site des amorces pour le clonage et le séquençage des anticorps humains. Séquence consensus des parties constantes humaines Igλ avec l'addition du site de restriction *Mlu* I*.*

### EXEMPLE 1

La figure 1 représente (en ligne pointillée) un brin d'ARN « sens » codant pour la chaîne lourde d'un anticorps, ledit ARN ayant été récupéré à partir d'un hybridome, pour cet exemple. Comme cela a été préalablement décrit, ce brin d'ARN a été purifié. Ce brin d'ARN comprend, dans le sens 5'3' la séquence codant pour la région variable V ainsi que la séquence codant pour la région constante C. Plus particulièrement, la région variable V est constituée du motif 5'-UTR, du peptide signal (Ps) ainsi que des gènes v, d et j. Le gène v correspond au gène de variabilité, le gène d au gène de diversité et le gène j au gène de jonction.

Dans le cas de la chaîne légère, le brin d'ARN « sens » est dépourvu de gène d. Dans la présente demande de brevet, seul l'exemple de la chaîne lourde sera décrit en détail, le cas de la chaîne légère étant identique au niveau des différentes étapes du procédé.

Une amorce primaire « anti-sens » (I) est hybridée au niveau de la région constante C. Plus particulièrement, selon une forme d'exécution préférée représentée ici, l'amorce primaire (I) vient s'hybrider à proximité de l'extrémité 5' de la séquence codant pour la région constante C.

Comme cela a été décrit plus haut, ce point présente un avantage particulièrement intéressant pour l'invention en ce sens qu'il permet d'assurer que, lors de la synthèse d'un brin d'ADNc, l'intégralité des gènes v, d et j sera bien conservée.

A partir de ladite amorce primaire (I), est synthétisé un brin d'ADNc « anti-sens » (représenté en ligne continue) qui comprend, quant à lui, dans le sens 5'3' les séquences complémentaires des gènes j, d et v.

L'hybride d'ADNc/ARN est ensuite traité avec une ARNase de manière à dégrader le brin d'ARN et obtenir un simple brin d'ADNc qui puisse être circularisé par la suite.

L'étape de circularisation est représentée à la figure 2.

Plus particulièrement, est représenté le brin d'ADNc, comprenant toujours dans le sens 5'3' la séquence codant pour la portion de la région constante C comprenant l'amorce (I) et la séquence correspondant à la région variable, cette dernière comprenant les gènes j, d et v, le tout étant circularisé par liaison covalente des extrémités 5' et 3' entre elles. Pour ce faire, comme cela ressortira entre autre de l'exemple ci-dessous, l'extrémité 5' comprend de manière classique un groupement phosphate (PO₄-) alors que l'extrémité 3' comprend, quant à elle, un groupement hydroxyde (OH).

La figure 3 représente les quatre premiers tours d'amplification en « cercles tournants » ou « en cercles roulants » (d'après la nomenclature anglaise « *rolling circle* »).

La figure 3a) représente le premier tour d'amplification. L'amorce secondaire « sens » (II) s'hybride à l'ADNc circulaire, au niveau de la séquence comprise entre l'extrémité 5' de l'amorce primaire (I) et l'extrémité 3' de la région constante C. Dans la forme d'exécution représentée ici, l'amorce secondaire (II) s'hybride au niveau de l'extrémité 5' de l'amorce primaire (I). Il est bien évident que cette position d'hybridation n'est pas limitative, mais n'est représentée ici qu'à titre illustratif. La synthèse du brin complémentaire d'ADN « sens » (représenté en ligne pointillée) est alors réalisée par ajout des nucléotides libres présents dans le milieu de réaction à partir de ladite amorce secondaire (II), dans le sens 5'3'. La fin de ce premier tour d'amplification débouche sur l'obtention d'une première unité (1) d'ADN « sens » hybridé à l'ADNc circulaire.

La figure 3b) représente, de manière schématique, le tour d'amplification suivant. Dans la continuité du premier tour d'amplification, un second tour d'amplification est mis en oeuvre au cours duquel les nucléotides libres présents dans le milieu de réaction sont ajoutés au niveau de l'extrémité 3' de la première unité (1) d'ADN « sens » formant ainsi une deuxième unité (2) d'ADN « sens » qui, lors de sa synthèse, va venir prendre la place de la première unité 1 le long de l'ADNc circulaire en déplaçant cette dernière. Il en résulte une molécule d'ADN constituée de la deuxième unité (2) d'ADN « sens » hybridée à l'ADNc et solidaire, au niveau de son extrémité 5', de la première unité (1) d'ADN « sens » déplacée et donc, de ce fait, simple brin. Simultanément, au moment où la première unité (1) d'ADN linéaire synthétisée est déplacée par la polymérase de la deuxième unité (2), la séquence correspondant au site d'hybridation de l'amorce tertiaire (III) est libérée (c'est-à-dire qu'elle est simple brin). L'amorce tertiaire (III) vient alors s'hybrider et initier la synthèse d'un premier brin d'ADN « anti-sens » 11(représenté en ligne continue) complémentaire de la première unité (1) d'ADN « sens ».

La figure 3c) représente un tour suivant du procédé d'amplification.

Le même mécanisme se produit, entraînant la synthèse d'une troisième unité (3) d'ADN « sens » le long de l'ADNc circulaire, déplaçant ainsi le segment d'ADN « sens » constitué des première et deuxième unités (1, 2) ladite première unité (1) consistant en un ADN double brin du fait du tour d'amplification précédent alors que la deuxième unité (2) consiste en un simple brin d'ADN « sens ». De manière similaire à l'étape précédente, le déplacement de la deuxième unité (2) d'ADN « sens » va libérer le site d'hybridation de l'amorce tertiaire (III) qui va venir se fixer et initier la synthèse d'un deuxième brin d'ADN « anti-sens » (12) complémentaire de la deuxième unité (2) d'ADN « sens ». Lorsque ce deuxième brin d'ADN « anti-sens » (12) nouvellement synthétisé va venir buter contre l'extrémité 5' du premier brin d'ADN « anti-sens » (11) complémentaire de la première unité (1), il va alors prendre sa place le long de la séquence d'ADN « sens », libérant ainsi le premier brin d'ADN « anti-sens » (11) complémentaire de la première unité (1). L'amorce secondaire (II) viendra alors s'hybrider au site d'hybridation rendu ainsi libre au niveau de l'extrémité 3' et initiera la synthèse du brin complémentaire d'ADN « sens » suivant l'orientation 5'3'.

La figure 3d) représente le tour suivant d'amplification.

De manière similaire, une quatrième unité (4) d'ADN « sens » est synthétisée le long de l'ADNc circulaire, déplaçant ainsi le segment d'ADN constitué des trois premières unités (1, 2, 3) d'ADN « sens » résultant des tours d'amplification précédents.

A ce niveau du procédé, les première et deuxième unités (1, 2) consistent en un ADN double brin du fait des tours d'amplification précédents alors que la troisième unité (3) consiste en un simple brin d'ADN « sens ». De manière similaire à l'étape précédente, le déplacement de la troisième unité (3) d'ADN « sens » va libérer le site d'hybridation de l'amorce tertiaire (III) qui va venir se fixer et initier la synthèse d'un troisième brin d'ADN « anti-sens » (13) complémentaire de la troisième unité (3) d'ADN « sens ». Lorsque ce troisième brin d'ADN « anti-sens » nouvellement synthétisé va venir buter contre l'extrémité 5' du deuxième brin d'ADN « anti-sens » (12) complémentaire des première et deuxième unités (1, 2), il va alors prendre sa place le long de la séquence d'ADN «sens», libérant ainsi ledit deuxième brin d'ADN « anti-sens » (12) complémentaire des première et deuxième unités (1, 2). L'amorce secondaire (II) pourra alors venir s'hybrider au site d'hybridation rendu ainsi libre au niveau de l'extrémité 3' de la première unité (I) ou de la deuxième unité (2), et initiera la synthèse d'un brin d'ADN « sens » complémentaire des première et deuxième unités (1, 2), selon l'orientation 5'3'. Dans le cas de figure où l'amorce secondaire est venue s'hybrider au niveau de l'extrémité 3' de la deuxième unité (2), la synthèse d'un brin d'ADN « sens » complémentaire n'a pu être réalisée que pour la deuxième unité (2). Il s'en suit que, par la suite, l'amorce secondaire (II) peut venir s'hybrider au niveau de l'extrémité 3' de la première unité (1), initiant ainsi la synthèse d'un brin d'ADN « sens » complémentaire de la première et de la deuxième unité (1, 2). Or, au niveau de cette deuxième unité (2), la synthèse du brin complémentaire d'ADN « sens » va venir déplacer et libérer le brin d'ADN « sens » précédemment synthétisé. L'amorce secondaire (II) pourra alors venir s'hybrider à ce brin d'ADN « sens » libéré et initiera la synthèse du brin d'ADN « anti-sens » complémentaire (non représenté).

### EXEMPLE 2 : Clonage et séquencage par approche « indirecte » des gènes d'un anticorps à partir d'un hybridome

### I. Purification des ARN

Les ARN totaux sont isolés à partir de 5 x 10⁶ cellules d'un hybridome préalablement concentrés par centrifugation et congelés sous forme de culot de cellules à -80°C. Le culot de cellules est décongelé puis est dénaturé en vue de l'isolation des ARN. Le Mini Kit RNeasy® (Qiagen) est utilisé pour isoler les ARN totaux. La purification des ARN totaux est réalisée selon les instructions du fournisseur. Le culot de cellules est lysé par addition de 600 µl de tampon RLT contenant du béta-mercaptoéthanol puis est ensuite homogénéisé en passant 6 fois au travers d'une aiguille d'une épaisseur de 20 gauges. Un volume de 600 µl d'éthanol à 70 % est ajouté au lysat homogénéisé puis le tout est mélangé par pipetage. Le lysat est ensuite appliqué à la mini-colonne RNeasy en deux aliquots de 600 µl chacun. Une centrifugation à 10.000 g pendant 15 secondes est effectuée après chaque application. La mini-colonne RNeasy est lavée avec 500 µl de tampon RPE (Qiagen) puis centrifugée à 10.000 g pendant 15 secondes. La mini-colonne RNeasy est ensuite transférée dans un nouveau tube microcentrifuge, lavée avec 500 µl supplémentaires de tampon RPE puis centrifugée à 10.000 g pendant 2 minutes. La mini-colonne RNeasy est ensuite placée dans un nouveau tube microcentrifuge et 50 µl d'eau sans RNase/DNase (Ambion) sont ajoutés à la colonne. La mini-colonne RNeasy est de nouveau centrifugée à 10.000 g pendant 1 minute. Les ARN totaux purifiés sont ensuite quantifiés par spectrophotométrie à 260/280 nm et sont ensuite immédiatement utilisés pour la synthèse des ADNc.

### II. Synthèse et purification des ADNc

La synthèse des ADNc est réalisée avec la transcriptase reverse Superscript III (Invitrogen) en utilisant les ARN totaux comme matrice et l'amorce spécifique du gène de la chaîne lourde CHssDNA (5' PO₄- AGC AGA CCC GGG GGC CAG TGG ATA GAC AG 3', SEQ ID N° 1) ou l'amorce spécifique du gène de la chaîne légère VLssDNA (5' PO₄- TCC AGA TGT TAA CTG CTC ACT GGA TGG TGG GAA GAT GGA TAC AG 3', SEQ ID N° 2). Les séquences des amorces sont choisies de manière à comprendre nativement ou à permettre l'inclusion de sites de restriction par des modifications mineures de la séquence mère. La séquence de l'amorce de la chaîne lourde est modifiée de manière à inclure le site de restriction *Xma* I. La séquence de l'amorce de la chaîne légère comprend, quant à elle, le site de restriction natif *Hpa* I. La synthèse du premier brin d'ADNc est réalisée avec 2,5 µg d'ARN totaux, 1 µl dNTP mix (10 mM de chaque, Ozyme), 2 µl d'amorce spécifique du gène (1 µM, 5' phosphorylé, Eurogentec), le tout étant complété avec de l'eau sans DNase/RNase (Ambion) jusqu'à un volume total de 14 µl. Le mélange ainsi obtenu est chauffé à 65°C pendant 5 minutes puis est ensuite refroidi sur de la glace pour permettre l'hybridation des amorces sur la matrice. Sont ensuite ajoutés audit mélange : 4 µl de 5x « 1st strand buffer » «(Invitrogen), 1 µl de DTT (1 M) et 1 µl de Superscript III (200 U/µl). Le mélange de réaction est ensuite incubé à 55°C pendant 1 heure et les enzymes sont inactivées par chauffage à 70°C pendant 15 minutes.

La synthèse du premier brin finalisée, ce dernier est ensuite traité avec le kit de purification PCR (Qiagen) pour éliminer les amorces non incorporées. La synthèse d'ADNc est complétée par ajout de 50 µl d'eau sans DNase/RNase auxquels sont ajoutés 250 µl de tampon PB (Qiagen) et la solution est mélangée par pipetage. Le mélange est ensuite transféré sur une colonne microcentrifuge PCR Purification Kit puis est centrifugé à 10.000 g pendant 1 minute. La colonne est lavée avec 750 µl de tampon PE (Qiagen) puis est ensuite centrifugée à 10.000 g pendant 1 minute. La colonne est de nouveau centrifugée à 10.000 g pendant 1 minute. Elle est ensuite transférée dans un nouveau tube microcentrifuge de 1,5 ml et 30 µl de tampon EB (10 mM Tris-HCl pH8.0, Qiagen) sont ajoutés au centre de la colonne pour éluer l'ADNc puis l'ensemble est de nouveau centrifugé à 10.000 g pendant 1 minute. L'ADNc récupéré est ensuite stocké à -20°C.

D'autres étapes successives à la synthèse d'ADNc comme le traitement à la RNase peuvent être réalisées mais ne sont pas essentielles.

### III. Circularisation de l'ADNc

L'ADNc simple brin est circularisé à l'aide de la ligase CircLigase™ (Epicentre) qui introduit une liaison covalente entre l'extrémité 3' OH et l'extrémité 5' PO₄ de l'ADNc. Le groupement PO₄ a été préalablement introduit au niveau de l'extrémité 5' lors de la synthèse de l'amorce spécifique du gène qui a été utilisée pour la synthèse de l'ADNc. La réaction de circularisation est réalisée avec 16 µl d'ADNc purifié, 2 µl de tampon de réaction 10x CircLigase (Epicentre), 1 µl d'ATP (1 mM), 1 µl de CircLigase (100 U/µl). La réaction est ensuite incubée à 60°C pendant 1 heure puis les enzymes sont inactivées par incubation à 80°C pendant 10 minutes. Suite à la réaction de circularisation, le mélange obtenu est complété jusqu'à 50 µl avec de l'eau sans DNase/RNase auxquels sont additionnés 250 µl de tampon PB (Qiagen) et la solution est mélangée par pipetage. Le mélange est ensuite transféré sur une colonne microcentrifuge PCR Purification Kit et centrifugé à 10.000 g pendant 1 minute. La colonne est lavée avec 750 µl de tampon PE (Qiagen) puis est ensuite de nouveau centrifugée à 10.000 g pendant 1 minute. La colonne est centrifugée une nouvelle fois à 10.000 g pendant 1 minute. Elle est ensuite transférée dans un nouveau tube microcentrifuge de 1,5 ml et 30 µl de EB (10 mM Tris-HCl pH8.0, Qiagen) sont ajoutés au centre de la colonne pour éluer l'ADNc et l'ensemble est de nouveau centrifugé à 10.000 g pendant 1 minute. L'ADNc circularisé récupéré est ensuite stocké à -20°C.

### IV. Amplification par « cercle tournant »

L'ADNc simple brin circularisé est amplifié en utilisant le kit d'amplification illustra TempliPhi^{™} (Amersham Biosciences) et les amorces spécifiques du gène homologue des séquences des amorces utilisées pour la synthèse de l'ADNc. La réaction d'amplification est réalisée avec 5 µl de tampon TempliPhi, 0,5 µl d'ADNc circularisé et 0,25 µl d'amorces sens et anti-sens (100 µM, Sigma ProOligo). Les amorces sont illustrées dans le tableau 1 ci-dessous.

**Tableau 1**

| | Chaîne lourde | Chaîne légère |
|---|---|---|
| Anti-sens (I) | | |
| Sens (II) | 5'-CCACTGGCCCCCGGGTCTGC-3' SEQ ID N° 3 | 5'-GTGAGCAGTTAACATCTGG-3' SEQ ID N° 4 |
| Anti-sens (III) | 5'-GATAGACAGATGGGGGTGTCG-3' SEQ ID N° 5 | 5'-GGTGGGAAGATGGATACAG-3' SEQ ID N° 6 |

Les amorces sens et anti-sens sont synthétisées avec des liaisons phosphorothioates de manière à les protéger de l'activité exonucléase de la polymérase φ29. Le mélange de réaction est chauffé à 95°C pendant 3 minutes et ensuite refroidi sur de la glace. Un second mélange est préparé avec 5 µl de tampon de réaction TempliPhi et 0,2 µl d'enzyme TempliPhi. Cinq µl de ce mélange sont ensuite ajoutés au premier mélange de réaction préparé antérieurement. Le mélange finalisé est incubé à 30°C pendant 18 heures puis les enzymes sont inactivées à 65°C pendant 10 minutes. L'ADN double brin amplifié est stocké à -20°C.

### V. Clonage du gène de l'anticorps amplifié

Après l'amplification, le concatémère d'ADN double brin est digéré avec les enzymes de restriction compatibles avec les amorces oligonucléotidiques utilisées pour la synthèse de l'ADNc. Les chaînes lourdes des immunoglobulines amplifiées sont digérées comme suit : 7,5 µl d'ADN amplifié, 0,5 µl de BSA 20x, 1 µl de tampon 4 10x (NEB), 1 µl de *Xma* I (NEB). Le mélange est incubé à 37°C pendant 4 heures puis les enzymes sont inactivées à 65°C pendant 20 minutes. Les chaînes légères des immunoglobulines amplifiées sont digérées comme suit : 7,5 µl d'ADN amplifié, 0,5 µl d'eau, 1 µl de tampon 4 10x (NEB), 1 µl de *Hpa* I (NEB). Le mélange est incubé à 37°C pendant 4 heures. Les chaînes lourdes et légères digérées sont alors clonées respectivement dans les vecteurs de séquençage pUC18 et pGEM-T. Les inserts d'ADN clonés sont alors séquencés avec le «BigDye Terminator v3.1 Cycle Sequencing Kit»(Applied Biosystems) et les amorces correspondant aux vecteurs de séquençage.

La séquence obtenue après le séquençage pour la chaîne lourde totale est représentée à la figure 6 (SEQ ID N° 11). La portion correspondant au domaine variable, séquence d'acides aminés en gras sur la figure 6, est représentée à la séquence SEQ ID N° 7 alors que la séquence correspondant au peptide signal, double souligné sur la figure 6, est représentée à la séquence SEQ ID N° 9.

La séquence obtenue après le séquençage pour la chaîne légère totale est représentée à la figure 9 (SEQ ID N° 12). La portion de l'acide nucléique correspondant au domaine variable, séquence d'acides aminés en gras sur la figure 9, est représentée à la séquence SEQ ID N° 13.

### VI. Analyse comparative des séquences du domaine variable et du peptide signal de la chaîne lourde de l'anticorps obtenues par une méthode classique et par l'approche objet de l'invention

L'approche classique d'obtention des séquences des domaines variables d'un anticorps à partir d'un hybridome consiste en l'amplification par PCR desdits domaines variables à l'aide d'amorces situées dans le peptide signal, pour l'amorce sens, et en 5' du domaine constant CH1 pour la chaîne lourde et Cκ pour la chaîne légère, pour l'amorce anti-sens. Il est à noter que l'amorce sens utilisée correspond habituellement à une séquence dégénérée de façon à pouvoir s'hybrider à toutes les séquences supposées possibles et connues des peptides signaux naturellement utilisés par l'organisme d'où provient ledit anticorps.

La séquence obtenue de manière classique pour le domaine variable de la chaîne lourde est représentée à la séquence SEQ ID N° 8.

Les séquences obtenues pour le domaine variable de la chaîne lourde de l'anticorps exemplifié sont ensuite caractérisées en réalisant une analyse immunogénétique selon la nomenclature IMGT qui permet en outre d'identifier les réarrangements des différents gènes mis en jeux et de rechercher dans la base de donnée la séquence génomique du peptide signal associée aux gènes V utilisés lors de la génération des domaines variables de l'anticorps exemplifié.

Le tableau 2 ci-dessous montre les résultats obtenus pour la chaîne lourde.

**Tableau 2**

| Séquence | Gène V | % Identité | Gène J | Gène D | CDRs-IMGT | Jonction VxDxJ |
|---|---|---|---|---|---|---|
| Approche classique | IGHV1-18*01 | 96,88% (279/288 nt) | IGHJ2*01 | IGHD2-4*01 | [12, 10, 11] | CARREITTEFDYW |
| approche inventive | IGHV1-13*01 | 96,88% (279/288 nt) | ICHJ2*01 | IGHD2-4*01 | [12, 10, 11] | CARREITTEFDYW |

L'alignement des séquences, représenté à la figure 7, aussi bien que l'analyse immunogénétique, confirment l'identité existant entre les séquences du domaine variable de la chaîne lourde obtenues par l'approche selon l'invention (SEQ ID N° 7) et par l'approche classique (SEQ ID N° 8).

La même démarche a été réalisée pour les séquences correspondant au peptide signal, à savoir la séquence obtenue avec le procédé selon l'invention (SEQ ID N° 9) et obtenue selon l'approche classique (SEQ ID N° 10).

La recherche dans la base de séquences IMGT permet d'obtenir les informations relatives à l'identification du gène V IGHV1-18*01 (IMGT Flat File) et d'en extraire la séquence nucléique et protéique du peptide signal associé au gène. En l'occurrence l'allèle IGHV1-18*01 identifié comme le plus proche de la séquence du domaine variable de la chaîne lourde de l'anticorps exemplifié a été identifié à partir de la souche de souris C57BL/6J. Dans l'exemple, la souche de souris utilisée pour générer l'anticorps est BALB/c pour lequel la lignée germinale existe sous l'allèle IGHV1-18*02^{.} L'alignement de la séquence obtenue pour le domaine variable de la chaîne de l'anticorps exemplifié montre une homologie 98,02 % (248/253 nt). Cette homologie est plus importante que celle identifiée pour l'allèle IGFIV1-18*01 mais ne concerne qu'une séquence raccourcie du gène V à savoir 253 nt pour l'allèle *02 chez BALB/c contre 288 nt pour l'allèle *01 chez C57BL/6J. Ceci expliquant pourquoi l'analyse initiale a favorisé l'allèle *01. En ce qui concerne l'identification du peptide signal, il est donc nécessaire de s'intéresser à la séquence retrouvée dans la souche de souris utilisée et correspondant à l'allèle IGHV1-18*02.

L'alignement des séquences correspondant au peptide signal, représenté à la figure 8, montre clairement que la méthode de la présente invention permet d'obtenir la séquence génomique (SEQ ID N° 9) contrairement à l'approche classique qui introduit un biais dans la séquence correspondante (SEQ ID N° 10), en l'occurrence dans cet exemple, au changement de 4 nucléotides induisant 4 mutations au niveau de la séquence protéique.

En conclusion, cet exemple permet de confirmer que les séquences obtenues correspondent bien non seulement à des lignées germinales d'anticorps mais surtout aux séquences obtenues par les méthodes classiques. En outre, cet exemple met également en évidence le fait que le procédé selon l'invention permet de conserver le peptide signal natif qui se trouve modifié dans les approches classiques.

### VII. Analyse comparative des séquences du domaine variable et du peptide signal de la chaîne légère de l'anticorps obtenues par une méthode classique et par l'approche objet de l'invention

Une approche similaire a été réalisée et les mêmes résultats (non représentés) ont été obtenus.

### EXEMPLE 3 : Clonage et séquençage par approche « indirecte » des gènes d'un anticorps à partir d'un hybridome

### I. Purification des ARN

Les ARN totaux sont isolés à partir de 5 x 10⁶ cellules d'un hybridome préalablement concentrés par centrifugation et congelés sous forme de culot de cellules à -80°C. Le culot de cellules est décongelé puis est dénaturé en vue de l'isolement des ARN. Le Mini Kit RNeasy® (Qiagen) est utilisé pour isoler les ARN totaux. La purification des ARN totaux est réalisée selon les instructions du fournisseur. Le culot de cellules est lysé par addition de 600 µl de tampon RLT contenant du béta-mercaptoéthanol puis est ensuite homogénéisé en passant 6 fois au travers d'une aiguille d'une épaisseur de 20 gauges. Un volume de 600 µl d'éthanol à 70 % est ajouté au lysat homogénéisé puis le tout est mélangé par pipetage. Le lysat est ensuite appliqué à la mini-colonne RNeasy en deux aliquots de 600 µl chacun. Une centrifugation à 10.000 g pendant 15 secondes est effectuée après chaque application. La mini-colonne RNeasy est lavée avec 500 µl de tampon RPE (Qiagen) puis centrifugée à 10.000 g pendant 15 secondes. La mini-colonne RNeasy est ensuite transférée dans un nouveau tube microcentrifuge, lavée avec 500 µl supplémentaires de tampon RPE puis centrifugée à 10.000 g pendant 2 minutes. La mini-colonne RNeasy est ensuite placée dans un nouveau tube microcentrifuge et 50 µl d'eau sans RNase/DNase (Ambion) sont ajoutés à la colonne. La mini-colonne RNeasy est de nouveau centrifugée à 10.000 g pendant 1 minute. Les ARN totaux purifiés sont ensuite quantifiés par spectrophotométrie à 260/280 nm et sont ensuite immédiatement utilisés pour la synthèse des ADNc.

### II. Synthèse et purification des ADNc

La synthèse des ADNc est réalisée avec la transcriptase reverse Superscript III (Invitrogen) en utilisant les ARN totaux comme matrice et l'amorce spécifique du gène de la chaîne lourde IGHC1pO (5' PO₄- ACA AAC GCG TAT AGC CCT TGA CCA GGC ATC C 3', SEQ ID N° 14) ou l'amorce spécifique du gène de la chaîne légère IGKpO (5' PO₄- ACA AAC GCG TTG GTG GGA AGA TGG ATA CAG 3', SEQ ID N° 15). Les séquences des amorces comprennent le site de restriction Mlu **I** qui a été ajouté au niveau de l'extrémité 5' de la séquence native. La synthèse du premier brin d'ADNc est réalisée avec 2,5 µg d'ARN totaux, 1 µl dNTP mix (10 mM de chaque, Ozyme), 1 µl d'amorce spécifique du gène (50 µM, 5' phosphorylé, Eurogentec), le tout étant complété avec de l'eau sans DNase/RNase (Ambion) jusqu'à un volume total de 10 µl. Le mélange ainsi obtenu est chauffé à 65°C pendant 5 minutes puis est ensuite refroidi sur de la glace pour permettre l'hybridation des amorces sur la matrice. Sont ensuite ajoutés audit mélange : 4 µl de 5x « Prime Script Buffer » (Takara), 0,5 µl de Rnase inhibitor (40 U/µl), 1 µl de Prime Script RTase (200 U/µl) et le volume est ensuite complété avec 20 µl de H₂O. Le mélange de réaction est ensuite incubé à 50°C pendant 1 heure et les enzymes sont inactivées par chauffage à 70°C pendant 15 minutes.

La synthèse du premier brin finalisée, ce dernier est ensuite traité avec le kit de purification PCR (Qiagen) pour éliminer les amorces non incorporées. La synthèse d'ADNc est complétée par ajout de 50 µl d'eau sans DNase/RNase auxquels sont ajoutés 250 µl de tampon PB (Qiagen) et la solution est mélangée par pipetage. Le mélange est ensuite transféré sur une colonne microcentrifuge PCR Purification Kit puis est centrifugé à 10.000 g pendant 1 minute. La colonne est lavée avec 750 µl de tampon PE (Qiagen) puis est ensuite centrifugée à 10.000 g pendant 1 minute. La colonne est de nouveau centrifugée à 10.000 g pendant 1 minute. Elle est ensuite transférée dans un nouveau tube microcentrifuge de 1,5 ml et 30 µl de tampon EB (10 mM Tris-HCl pH8.0, Qiagen) sont ajoutés au centre de la colonne pour éluer l'ADNc puis l'ensemble est de nouveau centrifugé à 10.000 g pendant 1 minute. L'ADNc récupéré est ensuite stocké à -20°C.

D'autres étapes successives à la synthèse d'ADNc comme le traitement à la RNase peuvent être réalisées mais ne sont pas essentielles.

### III. Circularisation de l'ADNc

L'ADNc simple brin est circularisé à l'aide de la ligase CircLigase^{™} (Epicentre) qui introduit une liaison covalente entre l'extrémité 3' OH et l'extrémité 5' PO₄ de l'ADNc. Le groupement PO₄ a été préalablement introduit au niveau de l'extrémité 5' lors de la synthèse de l'amorce spécifique du gène qui a été utilisée pour la synthèse de l'ADNc. La réaction de circularisation est réalisée avec 1 µl d'ADNc purifié, 1 µl de tampon de réaction 10x CircLigase (Epicentre), 1 µl d'ATP (1 mM), 1 µl de CircLigase (100 U/µl) et 6 µl H₂O. La réaction est ensuite incubée à 60°C pendant 1 heure puis les enzymes sont inactivées par incubation à 80°C pendant 10 minutes. Suite à la réaction de circularisation, le mélange obtenu est complété jusqu'à 50 µl avec de l'eau sans DNase/RNase auxquels sont additionnés 250 µl de tampon PB (Qiagen) et la solution est mélangée par pipetage. Le mélange est ensuite transféré sur une colonne microcentrifuge PCR Purification Kit et centrifugé à 10.000 g pendant 1 minute. La colonne est lavée avec 750 µl de tampon PE (Qiagen) puis est ensuite de nouveau centrifugée à 10.000 g pendant 1 minute. La colonne est centrifugée une nouvelle fois à 10.000 g pendant 1 minute. Elle est ensuite transférée dans un nouveau tube microcentrifuge de 1,5 ml et 30 µl de EB (10 mM Tris-HCl pH 8.0, Qiagen) sont ajoutés au centre de la colonne pour éluer l'ADNc et l'ensemble est de nouveau centrifugé à 10.000 g pendant 1 minute. L'ADNc circularisé récupéré est ensuite stocké à -20°C.

### IV. Amplification par « cercle tournant »

L'ADNc simple brin circularisé est amplifié en utilisant le kit d'amplification illustra TempliPhi^{™} (Amersham Biosciences) et les amorces spécifiques du gène homologue des séquences des amorces utilisées pour la synthèse de l'ADNc. La réaction d'amplification est réalisée avec 4 µl de H₂O, 0,5 µl d'ADNc circularisé et 0,5 µl d'amorces sens et anti-sens (100 µM, Sigma ProOligo). Les amorces sont illustrées dans le tableau 3 ci-dessous.

**Tableau 3**

| | Chaîne lourde | Chaîne légère |
|---|---|---|
| Anti-sens (I) | | |
| Sens (II) | 5'- CTAACTCCATGGTGACCCTG -3' SEQ ID N° 16 | 5'- GGGCTGATGCTGCACCAAC -3' SEQ ID N° 17 |
| Anti-sens (III) | 5'-GATAGACAGATGGGGGTGTCG-3' SEQ ID N° 5 | 5'-GGTGGGAAGATGGATACAG-3' SEQ ID N° 6 |

Les amorces sens et anti-sens sont synthétisées avec des liaisons phosphorothioates de manière à les protéger de l'activité exonucléase de la polymérase φ29. Le mélange de réaction est chauffé à 95°C pendant 3 minutes et ensuite refroidi sur de la glace. Un second mélange est préparé avec 5 µl de tampon de réaction TempliPhi et 0,2 µl d'enzyme TempliPhi. 5 µl de ce mélange sont ensuite ajoutés au premier mélange de réaction préparé antérieurement. Le mélange finalisé est incubé à 30°C pendant 18 heures puis les enzymes sont inactivées à 65°C pendant 10 minutes. L'ADN double brin amplifié est stocké à -20°C.

### V. Clonage du gène de l'anticorps amplifié

Après l'amplification, le concatémère d'ADN double brin est digéré avec les enzymes de restriction compatibles avec les amorces oligonucléotidiques utilisées pour la synthèse de l'ADNc. Les chaînes lourdes des immunoglobulines amplifiées sont digérées comme suit : 8 µl d'ADN amplifié, 1 µl de tampon 3 10x (NEB), 1 µl de *Mlu* 1 (NEB). Le mélange est incubé à 37°C pendant 4 heures puis les enzymes sont inactivées à 65°C pendant 20 minutes. Les chaînes légères des immunoglobulines amplifiées sont digérées comme suit : 8 µl d'ADN amplifié, 1 µl de tampon 3 10x (NEB), 1 µl de *Mlu* I (NEB). Le mélange est incubé à 37°C pendant 4 heures puis les enzymes sont inactivées à 65°C pendant 20 minutes. Les chaînes lourdes et légères digérées sont alors clonées respectivement dans le vecteur de séquençage pGEM-T. Les inserts d'ADN clonés sont alors séquencés avec le « BigDye Terminator v3.1 Cycle Sequencing Kit » (Applied Biosystems) et les amorces correspondant aux vecteurs de séquençage.

La séquence obtenue après le séquençage pour la chaîne lourde totale est représentée à la figure 10 (SEQ ID N° 18). La portion d'acide nucléique correspondant au domaine variable, séquence d'acides aminés en gras sur la figure 10, est représentée à la séquence SEQ ID N° 19.

### EXEMPLE 4 : Clonage et séquençage par approche « combinée » des gènes d'un anticorps à partir d'un hybridome

### I. Purification des ARN

Les ARN totaux sont isolés à partir de 5 x 10⁶ cellules d'un hybridome préalablement concentrés par centrifugation et congelés sous forme de culot de cellules à -80°C. Le culot de cellules est décongelé puis est dénaturé en vue de l'isolation des ARN. Le Mini Kit RNeasy® (Qiagen) est utilisé pour isoler les ARN totaux. La purification des ARN totaux est réalisée selon les instructions du fournisseur. Le culot de cellules est lysé par addition de 600 µl de tampon RLT contenant du béta-mercaptoéthanol puis est ensuite homogénéisé en passant 6 fois au travers d'une aiguille d'une épaisseur de 20 gauges. Un volume de 600 µl d'éthanol à 70 % est ajouté au lysat homogénéisé puis le tout est mélangé par pipetage. Le lysat est ensuite appliqué à la mini-colonne RNeasy en deux aliquots de 600 µl chacun. Une centrifugation à 10.000 g pendant 15 secondes est effectuée après chaque application. La mini-colonne RNeasy est lavée avec 500 µl de tampon RPE (Qiagen) puis centrifugée à 10.000 g pendant 15 secondes. La mini-colonne RNeasy est ensuite transférée dans un nouveau tube microcentrifuge, lavée avec 500 µl supplémentaires de tampon RPE puis centrifugée à 10.000 g pendant 2 minutes. La mini-colonne RNeasy est ensuite placée dans un nouveau tube microcentrifuge et 50 µl d'eau sans RNase/DNase (Ambion) sont ajoutés à la colonne. La mini-colonne RNeasy est de nouveau centrifugée à 10.000 g pendant 1 minute. Les ARN totaux purifiés sont ensuite quantifiés par spectrophotométrie à 260/280 nm et sont ensuite immédiatement utilisés pour la synthèse des ADNc.

### II Synthèse et purification des ADNc

La synthèse des ADNc est réalisée avec la transcriptase reverse Superscript III (Invitrogen) en utilisant les ARN totaux comme matrice et l'amorce spécifique du gène de la chaîne lourde IGHC1pO (5' PO₄- ACA AAC GCG TAT AGC CCT TGA CCA GGC ATC C 3', SEQ ID N° 14) ou l'amorce spécifique du gène de la chaîne légère IGKpO (5' PO₄- ACA AAC GCG TTG GTG GGA AGA TGG ATA CAG 3', SEQ ID N° 15). Les séquences des amorces sont choisies de manière à comprendre nativement ou à permettre l'inclusion de sites de restriction par des modifications mineures de la séquence mère. Les séquences des amorces de la chaîne lourde et légère sont modifiées de manière à inclure le site de restriction *Mlu* I à l'extrémité 5'. La synthèse du premier brin d'ADNc est réalisée avec 2,5 µg d'ARN totaux, 1 µl dNTP mix (10 mM de chaque, Ozyme), 2 µl d'amorce spécifique du gène (50 µM, 5' phosphorylé, Eurogentec), le tout étant complété avec de l'eau sans DNase/RNase (Ambion) jusqu'à un volume total de 14 µl. Le mélange ainsi obtenu est chauffé à 65°C pendant 5 minutes puis est ensuite refroidi sur de la glace pour permettre l'hybridation des amorces sur la matrice. Sont ensuite ajoutés audit mélange : 4 µl de 5x « 1st strand buffer » «(Invitrogen), 1 µl de DTT (1 M) et 1 µl de Superscript III (200 U/µl). Le mélange de réaction est ensuite incubé à 55°C pendant 1 heure et les enzymes sont inactivées par chauffage à 70°C pendant 15 minutes.

La synthèse du premier brin finalisée, ce dernier est ensuite traité avec le kit de purification PCR (Qiagen) pour éliminer les amorces non incorporées. La synthèse d'ADNc est complétée par ajout de 50 µl d'eau sans DNase/RNase auxquels sont ajoutés 250 µl de tampon PB (Qiagen) et la solution est mélangée par pipetage. Le mélange est ensuite transféré sur une colonne microcentrifuge PCR Purification Kit puis est centrifugé à 10.000 g pendant 1 minute. La colonne est lavée avec 750 µl de tampon PE (Qiagen) puis est ensuite centrifugée à 10.000 g pendant 1 minute. La colonne est de nouveau centrifugée à 10.000 g pendant 1 minute. Elle est ensuite transférée dans un nouveau tube microcentrifuge de 1,5 ml et 30 µl de tampon EB (10 mM Tris-HCl pH8.0, Qiagen) sont ajoutés au centre de la colonne pour éluer l'ADNc puis l'ensemble est de nouveau centrifugé à 10.000 g pendant 1 minute. L'ADNc récupéré est ensuite stocké à -20°C.

D'autres étapes successives à la synthèse d'ADNc comme le traitement à la RNase peuvent être réalisées mais ne sont pas essentielles.

### III. Circularisation de l'ADNc

L'ADNc simple brin est circularisé à l'aide de la ligase CircLigase™ (Epicentre) qui introduit une liaison covalente entre l'extrémité 3' OH et l'extrémité 5' PO₄ de l'ADNc. Le groupement PO₄ a été préalablement introduit au niveau de l'extrémité 5' lors de la synthèse de l'amorce spécifique du gène qui a été utilisée pour la synthèse de l'ADNc. La réaction de circularisation est réalisée avec 16 µl d'ADNc purifié, 2 µl de tampon de réaction 10x CircLigase (Epicentre), 1 µl d'ATP (1 mM), 1 µl de CircLigase (100 U/µl). La réaction est ensuite incubée à 60°C pendant 1 heure puis les enzymes sont inactivées par incubation à 80°C pendant 10 minutes. Suite à la réaction de circularisation, le mélange obtenu est complété jusqu'à 50 µl avec de l'eau sans DNase/RNase auxquels sont additionnés 250 µl de tampon PB (Qiagen) et la solution est mélangée par pipetage. Le mélange est ensuite transféré sur une colonne microcentrifuge PCR Purification Kit et centrifugé à 10.000 g pendant 1 minute. La colonne est lavée avec 750 µl de tampon PE (Qiagen) puis est ensuite de nouveau centrifugée à 10.000 g pendant 1 minute. La colonne est centrifugée une nouvelle fois à 10.000 g pendant 1 minute. Elle est ensuite transférée dans un nouveau tube microcentrifuge de 1,5 ml et 30 µl de EB (10 mM Tris-HCl pH 8.0, Qiagen) sont ajoutés au centre de la colonne pour éluer l'ADNc et l'ensemble est de nouveau centrifugé à 10.000 g pendant 1 minute. L'ADNc circularisé récupéré est ensuite stocké à -20°C.

### IV. Amplification par « cercle tournant »

L'ADNc simple brin circularisé est amplifié en utilisant le kit d'amplification illustra TempliPhi™ (Amersham Biosciences) et les amorces spécifiques du gène homologue des séquences des amorces utilisées pour la synthèse de l'ADNc. La réaction d'amplification est réalisée avec 5 µl d'eau, 0,5 µl d'ADNc circularisé et 0,25 µl d'amorces sens et anti-sens (100 µM, Sigma ProOligo). Les amorces étant identiques que celles de l'exemple 3 ci-dessus, elles sont illustrées dans le tableau 3.

Les amorces sens et anti-sens sont synthétisées avec des liaisons phosphorothioates de manière à les protéger de l'activité exonucléase de la polymérase φ29. Le mélange de réaction est chauffé à 95°C pendant 3 minutes et ensuite refroidi sur de la glace. Un second mélange est préparé avec 5 µl de tampon de réaction TempliPhi et 0,2 µl d'enzyme TempliPhi. Cinq µl de ce mélange sont ensuite ajoutés au premier mélange de réaction préparé antérieurement. Le mélange finalisé est incubé à 30°C pendant 18 heures puis les enzymes sont inactivées à 65°C pendant 10 minutes. L'ADN double brin amplifié est stocké à -20°C.

Les chaînes lourdes des immunoglobulines amplifiées sont digérées comme suit : 8 µl d'ADN amplifié, 1 µl de tampon 3 10x (NEB), 1 µl de *Mlu* I (NEB). Le mélange est incubé à 37°C pendant 4 heures puis les enzymes sont inactivées à 65°C pendant 20 minutes. Les chaînes légères des immunoglobulines amplifiées sont digérées comme suit : 8 µl d'ADN amplifié, 1 µl de tampon 3 10x (NEB), 1 µl de *Mlu* I (NEB). Le mélange est incubé à 37°C pendant 4 heures puis les enzymes sont inactivées à 65°C pendant 20 minutes. Les monomères de la chaîne lourde sont séparés par électrophorèse sur gel d'agarose (1 % p/v) à 10V/cm. L'ADN correspondant aux monomères est ensuite extrait et isolé avec le kit Nucleospin Extract II (Machery Nagel) selon le protocole du fabriquant avec une élution dans 15 µl de tampon « élution buffer ». L'ADN est alors séquencé avec le « BigDye Terminator v3.1 Cycle Sequencing Kit » (Applied Biosystems) en utilisant l'amorce primaire ou tertiaire selon la synthèse d'ADNc et par amplification par cercles roulants dans les conditions suivantes : 5 µl ADN monomerique, 2 µl 5x Tampon (Applied Biosystems), 2 µl BigDye Terminator v3.1, 1 µl d'amorce (10 µM). Les conditions de la réaction suivent la programmation de la machine Fast Thermal Cycler 9800 (Applied Biosystems) programme « BigDye std ».

La séquence obtenue après le séquençage pour la chaîne lourde totale est représentée à la figure 11 (SEQ ID N° 20). La portion d'acide nucléique correspondant au domaine variable, séquence d'acides aminés en gras sur la figure 11, est représentée à la séquence SEQ ID N° 21.

### EXEMPLE 5 : Comparaison des séquences obtenues par une approche « indirecte » et par une approche « combinée »

Afin de valider les différentes variantes de l'invention, à savoir l'approche indirecte et l'approche combinée (également appelée approche directe), il a été réalisé un alignement des séquences obtenues pour les chaînes lourdes totales respectivement par les deux approches.

La figure 12 illustre cet alignement.

Comme il ressort clairement de cette figure, les séquences obtenues sont identiques, ce qui valide les deux approches de la présente invention.

### EXEMPLE 6 : Mise en évidence de l'expression à la surface des cellules eucaryotes

Pour effectuer le criblage des anticorps clonés selon l'invention, un vecteur d'expression eucaryote a été modifié de manière à contenir un domaine transmembranaire (DTM) au niveau de l'extrémité C-terminal de la partie constante de la chaîne lourde d'un anticorps humain. Le DTM a été cloné en cis avec le domaine constant d'une chaîne lourde humaine de type IgG1 (Figure 13) de manière à ce que les anticorps exprimés dans les cellules hôtes n'aient plus à être exprimés dans le surnagent de culture mais soient retenus à la surface des cellules. Le clonage par restriction avec *Mlu* 1 et ligation dans le vecteur contenant le DTM permet l'insertion des parties variables comprenant leur portion 5'UTR et le peptide signal en cis avec le domaine constant, les chaînes légères sont clonées de la même manière dans un vecteur dépourvu de DTM (Figure 13). Les parties variables des chaînes lourdes et légères d'un anticorps murin ont été clonées dans les vecteurs d'expression en cis avec les domaines constants humains ; IgG 1 (chaîne lourde) ou Igk (chaîne légère), avec le DTM pour la chaîne lourde. Puis une co-transfection des cellules hôtes CHO avec les vecteurs d'expression de la chaîne lourde et légère a ensuite été effectuée par transfection avec la lipofectamine. Après un temps de croissance de 48 h dans du milieu de croissance avec sérum, les cellules ont été lavées avec du PBS.

La présence des anticorps sur la surface des cellules préalablement lavées, a été détectée par incubation des cellules avec les anticorps anti-humains IgG ou anti-souris IgG. Les anticorps anti-humains ou anti-souris IgG, préalablement marqués au flourophore ALEXA 488 (Molecular Probes, A11013), permettent l'identification des cellules qui portent les anticorps à la surface membranaire des cellules hôtes. Une analyse par cytométrie de flux permet alors la détection des cellules portant les anticorps à la surface par rapport aux cellules hôtes sans vecteur d'expression (Figure 14). La nature chimérique des anticorps, parties variables murines et parties constantes humaines, permet la détection avec les anticorps anti-humains IgG ou anti-souris IgG.

### EXEMPLE 7 : Listing des différentes amorces murines et humaines pouvant être utilisées dans le cadre de la mise en oeuvre de l'invention

Le présent exemple liste, à titre non limitatif, des séquences d'amorces préférées pour la mise en oeuvre de l'invention.

### I. Amorces murines

**Tableau 4 : Amorces primaires (I) pour la synthèse d'ADNc murin spécifique pour chaque isotype des domaines constant dans les souris Balb/c. Le site de restriction Mlu I est souligné et en italique.**

| Amorces | Séquence |
|---|---|
| IGHC1pO | ACAA*ACGCGT*ATAGCCCTTGACCAGGCATCC (SEQ ID No. 14) |
| IGHC2ApO | ACAA*ACGCGT*ATAACCCTTGACCAGGCATCC (SEQ ID No. 22) |
| IGHC2BpO | ACAA*ACGCGT*CAGGGATCCAGAGTTCCAAG (SEQ ID No. 23) |
| IGHC3pO | ACAA*ACGCGT*GTAGCCTTTGACAAGGCATCC (SEQ ID No. 24) |
| IGKpO | ACAA*ACGCGT*RGGTGGGAAGATGGATACAG (SEQ ID No. 15) |

**Tableau 5 : Amorces secondaires (II) pour l'amplification de l'ADNc circularisé. Les liens 3' sont des phosphothioate (*) à la place des phosphodiester.**

| Amorces | Séquence |
|---|---|
| IgCH1SPT | CTAACTCCATGGTGACCC*T*G (SEQ ID No. 16) |
| IgCH2aSPT | ACTGGCTCCTCGGTGAC*T*C (SEQ ID No. 25) |
| IgCH2bSPT | AGTGACTGTGACTTGGAAC*T*C (SEQ ID No. 26) |
| IgCH2bSbisPT | GTCTATCCACTGGCCCC*T*G (SEQ ID No. 27) |
| IgCH3SPT | CTGGATCCTCGGTGACAC*T*G (SEQ ID No. 28) |
| IgCKSPT | GGGCTGATGCTGCACCA*A*C (SEQ ID No. 17) |

**Tableau 6 : Amorces tertiaires (III) pour l'amplification de l'ADNc circularisé. Les liens 3' sont des phosphothioate (*) à la place des phosphodiester.**

| Amorces | Séquence |
|---|---|
| IgCH1ASPT | GATAGACAGATGGGGGTGT*C*G (SEQ ID No. 5) |
| IgCH2aASPT | GATAGACCGATGGGGC*T*G (SEQ ID No. 29) |
| IgCH2bASPT | GATAGACTGATGGGGGTGT*T*G (SEQ ID No. 30) |
| IgCH3ASPT | GATAGACAGATGGGGC*T*G (SEQ ID No. 31) |
| IgCKASPT | CAGTTGGTGCAGCATCA*G*C (SEQ ID No. 41) |

### II. Amorces humaines

**Tableau 7 : Amorces primaires (1) pour la synthèse d'ADNc humain spécifique pour tout isotype IgG. Le site de restriction Mlu I est souligné et en italique.**

| Amorces | Séquence |
|---|---|
| HuCHlpO | ACGCGTTTGACCAGGCAGCCCAGG (SEQ ID No. 32) |
| HuCκpO | ACGCGTCAGATTTCAACTGCTCATCAGATGG (SEQ ID No. 33) |
| HuCλpO | ACGCGTAGTGTGGCCTTGTTGGCTTG (SEQ ID No. 34) |

**Tableau 8 : Amorces secondaires (II) pour l'amplification de l'ADNc circularisé. Les liens 3' sont des phosphothioate (*) à la place des phosphodiester.**

| Amorces | Séquence |
|---|---|
| HuCHISPT | GCCCTGGGCTGCCTGG*T*C (SEQ ID No. 35) |
| HuCκSPT | CATCTGATGAGCAGTTGAAATCT*G*G (SEQ ID No. 36) |
| HUCλSPT | CAAGCCAACAAGGCCAC*A*C (SEQ ID No. 37) |

**Tableau 9 : Amorces tertiaires (III) pour l'amplification de l'ADNc circularisé. Les liens 3' sont des phosphothioate (*) à la place des phosphodiester.**

| Amorces | Séquence |
|---|---|
| HuCHIASPT | GATGGGCCCTTGGT*G*G (SEQ ID No. 38) |
| HuCκASPT | GGAAGATGAAGACAGATGGT*G*C (SEQ ID No. 39) |
| HUCλASPT | GGAGGGYGGGAACAGAGTG*A*C (SEQ ID No. 40) |

Les figures 15A, 15B et 15C illustrent également les différentes amorces et sites correspondants.

## Revendications

1. Procédé pour générer une séquence d'ADN codant pour une chaîne lourde ou une chaîne légère d'au moins un anticorps à partir d'un extrait ou d'un mélange d'ARN issu d'une cellule, cette cellule étant capable d'exprimer de l'ARN codant pour un anticorps ou pour la chaîne lourde et/ou la chaîne légère d'un anticorps, **caractérisé en ce que** ledit procédé comprend au moins les étapes suivantes :
a) la mise en contact d'une amorce primaire (I) avec ledit extrait ou mélange d'ARN susceptible de contenir au moins un ARN, dénommé ici ARN « sens », codant pour la chaîne lourde ou la chaîne légère d'un anticorps, cette amorce primaire étant capable de s'hybrider spécifiquement à un fragment de la séquence dudit ARN, « sens », ce fragment étant compris dans la séquence codant pour la région constante C de la chaîne lourde ou légère dudit anticorps ;
b) synthétiser, à partir de ladite amorce primaire (I), un ADNc simple brin dénommé ADNc « anti-sens » ;
c) éliminer, le cas échéant, les amorces primaires (I) n'ayant pas hybridé au fragment de la séquence dudit ARN à l'étape a) ;
d) circulariser ledit ADNc « anti-sens » par formation d'une liaison covalente entre son extrémité 5' et 3' ;
e) la mise en contact d'une amorce secondaire (II), dénommée amorce secondaire « sens », avec ledit ADNc circulaire « anti-sens » obtenu à l'étape d), cette amorce secondaire « sens » (In étant capable de s'hybrider audit ADNc circulaire « anti-sens » ;
f) amplifier ledit ADNc « anti-sens » à partir de ladite amorce secondaire (II) ; et
g) récupérer le brin d'ADN linéaire complémentaire « sens » ainsi amplifié.

2. Procédé selon la revendication 1, **caractérisé en ce que** ladite amorce primaire (I) est spécifique d'une séquence située à, ou adjacente à, l'extrémité 5' de la séquence d'ARN « sens » correspondant à la région constante C.

3. Procédé selon la revendication 1, **caractérisé en ce que** ladite amorce secondaire (II) est spécifique de la séquence comprise entre l'extrémité 5' de ladite séquence d'ADNc « anti-sens » et l'extrémité 3' de la séquence dudit ADNc « anti-sens » correspondant à la région constante C.

4. Procédé selon la revendication 3, **caractérisé en ce que** ladite amorce secondaire (II) est spécifique d'une séquence située à, ou adjacente à, l'extrémité 5' de la séquence d'ADNc « anti-sens ».

5. Procédé selon l'une quelconque des revendications 1 à 4, **caractérisé en ce que** lesdites amorces primaire (I) et secondaire (II) consistent chacune et indépendamment l'une de l'autre en une séquence d'ADN simple brin ayant une longueur comprise entre 10 et 100 nucléotides.

6. Procédé selon l'une des revendications 1 à 5, **caractérisé en ce qu'**il comprend, préalablement à l'étape a) d'hybridation de l'amorce primaire (I), une étape de dénaturation de l'ARN.

7. Procédé selon l'une des revendications 1 à 6, **caractérisé en ce que** ladite cellule capable de produire un anticorps consiste en un hybridome.

8. Procédé selon l'une des revendications 1 à 6, **caractérisé en ce que** ledit ARN est sélectionné parmi l'ARN issu de splénocytes, de nodules ou de lymphocytes B.

9. Procédé selon l'une des revendications 1 à 8, **caractérisé en ce que** ladite étape de circularisation est réalisée par mise en contact avec une ligase capable de lier par liaison covalente les extrémités 5' et 3' d'un ADN monobrin dénommée « ssDNA ligase ».

10. Procédé de séquençage d'une séquence d'ADN codant pour une chaîne lourde ou une chaîne légère d'au moins un anticorps à partir d'un extrait ou d'un mélange d'ARN issu d'une cellule, **caractérisé en ce qu'**il met en oeuvre un procédé pour générer une séquence d'ADN codant pour une chaîne lourde ou une chaîne légère d'au moins un anticorps selon l'une des revendications 1 à 9, et **en ce qu'**il comprend en outre, une étape de séquençage du brin d'ADN linéaire « sens » récupéré à l'étape g) dudit procédé de génération de la séquence d'ADN.

11. Procédé de génération d'une banque d'ADN codant pour au moins une chaîne lourde ou une chaîne légère d'au moins un anticorps à partir d'un extrait ou d'un mélange d'ARN issu d'une cellule, **caractérisé en ce qu'**il met en oeuvre un procédé pour générer une séquence d'ADN codant pour une chaîne lourde ou une chaîne légère d'au moins un anticorps selon l'une des revendications 1 à 10 et **en ce qu'**il comprend, en outre, les étapes suivantes :
h) la mise en contact d'une amorce tertiaire (III), dénommée amorce tertiaire « anti-sens », avec ledit ADN linéaire « sens » obtenu à l'étape g), cette amorce tertiaire « anti-sens » (III) étant capable de s'hybrider spécifiquement à un fragments de la séquence de l'ADN linéaire comprise entre l'extrémité 3' et l'extrémité 5' dudit brin d'ADN linéaire et correspondant à la séquence de cet ADN linéaire codant pour la région constante C ;
i) générer, à partir de ladite amorce tertiaire (III), un concatémère par synthèse du brin d'ADN « anti-sens » complémentaire, et
j) cloner ledit concatémère d'ADN double brin ainsi obtenu au sein de vecteurs préalablement préparés.

12. Procédé selon la revendication 11, **caractérisé en ce que** ladite amorce tertiaire (III) est spécifique d'une séquence située à, ou adjacente à, l'extrémité 5' de la séquence de l'ADN linéaire « sens » correspondant à la région constante C.

13. Procédé pour générer une banque d'ADN codant pour des chaînes lourdes ou légères d'anticorps selon la revendication 11, **caractérisé en ce qu'**il comprend préalablement à l'étape j), une étape consistant à segmenter le concatémère au niveau des séquences correspondant aux amorces utilisées.

14. Procédé selon la revendication 13, **caractérisé en ce que** l'amorce primaire (I) comprend un site de restriction.

15. Procédé selon la revendication 13 ou 14, **caractérisé en ce que** ladite étape j) de segmentation du concatémère est mise en oeuvre par digestion enzymatique à l'aide d'endonucléase de restriction spécifique du site de restriction compris dans l'amorce primaire (I).

16. Procédé selon lune des revendications 11 à 15, **caractérisé en ce que** le vecteur utilisé pour l'étape j) comprend en outre les séquences codant pour les domaines constants de la chaîne lourde ou légère d'une immunoglobuline.

17. Procédé selon la revendication 16, **caractérisé en ce que** ladite séquence codant pour le domaine constant de la chaîne lourde consiste préférentiellement en une séquence issue d'une immunoglobuline avec ancrage membranaire, ou comprenant une région C-terminale transmembranaire.

18. Procédé selon l'une des revendications 11 à 17, **caractérisé en ce qu'**il comprend, en outre, une étape k) de transfection avec le vecteur obtenu à l'étape j) d'une cellule hôte capable d'exprimer la chaîne lourde ou légère de l'anticorps codé par le fragment d'ADN double brin inséré au sein dudit vecteur.

19. Procédé selon la revendication 18, **caractérisé en ce que** ladite cellule hôte de l'étape k) est une cellule capable d'exprimer à sa surface l'anticorps codé par le fragment d'ADN double brin inséré.

20. Procédé selon la revendication 19, **caractérisé en ce que** ladite cellule hôte est une cellule eucaryote.

21. Utilisation d'un procédé selon l'une des revendications 1 à 20 pour la génération d'une librairie d'anticorps.

22. Procédé de screening *in vitro* d'anticorps actif contre une maladie donnée, **caractérisé en ce qu'**il met en oeuvre une étape dans laquelle le procédé selon l'une des revendications 18 à 20 est appliqué à un échantillon de sang préalablement prélevé d'un patient souffrant de ladite maladie donnée.

## Claims

1. A method for generating a DNA sequence coding for a heavy chain or a light chain of at least one antibody from an extract or mixture of RNA from a cell, this cell being capable of expressing the RNA coding for an antibody or for the heavy chain and/or the light chain of an antibody, **characterized in that** said method comprises at least the following steps:
a) putting a primary primer (I) in contact with said RNA extract or mixture liable to contain at least one RNA, designated here as a "sense" RNA, coding for the heavy chain or the light chain of an antibody, this primary primer being capable of specifically hybridizing to a fragment of the sequence of said "sense" RNA, this fragment being comprised in the sequence coding for the constant region C of the heavy or light chain of said antibody;
b) synthesizing, from said primary primer (I), a single strand cDNA designated as "anti-sense" cDNA;
c) eliminating if necessary, the primary primers (I) which have not hybridized to the fragment of the sequence of said RNA in step a);
d) annealing said "anti-sense" cDNA by forming a covalent bond between its 5' and 3'ends;
e) putting a secondary primer (II), designated as "sense" secondary primer, in contact with said "anti-sense" circular cDNA obtained in step d), this "sense" secondary primer (II) being capable of hybridizing to said "anti-sense" circular cDNA;
f) amplifying said "anti-sense" cDNA from said secondary primer (II); and
g) recovering the thereby amplified "sense" complementary linear DNA strand.

2. The method according to claim 1, **characterized in that** said primary primer (I) is specific of a sequence located at or adjacent to the 5'end of the sense RNA sequence corresponding to the constant region C.

3. The method according to claim 1, **characterized in that** said secondary primer (II) is specific of the sequence comprised between the 5' end of said "anti-sense" cDNA sequence and the 3'end of the sequence of said "anti-sense" cDNA corresponding to the constant region C.

4. The method according to claim 3, **characterized in that** said secondary primer (II) is specific of a sequence located at or adjacent to the 5'end of the "anti-sense" cDNA sequence.

5. The method according to any one of claims 1 to 4, **characterized in that** said primary (I) and secondary (II) primers each consist independently of each other in a sequence of single strand DNA having a length comprised between 10 and 100 nucleotides.

6. The method according to any one of claims 1 to 5, **characterized in that** it comprises, prior to step a) for hybridization of the primary primer (I), a step for denaturation of the RNA.

7. The method according to any one of claims 1 to 6, **characterized in that** said cell capable of producing an antibody consists in a hybridoma.

8. The method according to any one of claims 1 to 6, **characterized in that** said RNA is selected from RNA stemming from splenocytes, nodules or B lymphocytes.

9. The method according to any one of claims 1 to 8, **characterized in that** said annealing step is carried out by putting into contact with a ligase capable of binding through a covalent bond the 5' and 3' ends of a single strand DNA designated as "ssDNA ligase".

10. A method for sequencing a DNA sequence coding for a heavy chain or a light chain of at least one antibody from an extract or mixture of RNA from a cell, **characterized in that** it applies a method for generating a DNA sequence coding for a heavy chain or a light chain of at least one antibody according to any one of claims 1 to 9, and **in that** it further comprises a step for sequencing the strand of "sense" linear DNA recovered in step g) of said method for generating the DNA sequence.

11. A method for generating a bank of DNAs coding for at least one heavy chain or a light chain of at least one antibody from an extract or mixture of RNA from a cell, **characterized in that** it applies a method for generating a DNA sequence coding for a heavy chain or a light chain of at least one antibody according to any one of claims 1 to 10, and **in that** it further comprises the following steps:
h) putting a tertiary primer (III), designated as "anti-sense" tertiary primer, into contact with said "sense" linear DNA obtained in step g), this "anti-sense" tertiary primer (III) being capable of specifically hybridizing to a fragment of the sequence of the linear DNA comprised between the 3'end and the 5'end of said linear DNA strand and corresponding to the sequence of this linear DNA coding for the constant region C,
i) generating from said tertiary primer (III), a concatemer by synthesis of a complementary "anti-sense" DNA strand, and
j) cloning said thereby obtained double strand DNA concatemer within vectors prepared beforehand.

12. The method according to claim 11, **characterized in that** said tertiary primer (III) is specific of a sequence located at or adjacent to the 5'end of the sequence of the "sense" linear DNA corresponding to the constant region C.

13. A method for generating a bank of DNAs coding for heavy or light antibody chains according to claim 11, **characterized in that** it comprises, prior to step j), a step consisting of segmenting the concatemer at the sequences corresponding to the primers used.

14. The method according to claim 13, **characterized in that** the primary primer (I) comprises a restriction site.

15. The method according to claim 13 or 14, **characterized in that** said step j) for segmenting the concatemer is applied by enzymatic digestion with a specific restriction endonuclease of the restriction site comprised in the primary primer (I).

16. The method according to any one of claims 11 to 15, **characterized in that** the vector used for step j) further comprises sequences coding for the constant domains of the heavy or light chain of an immunoglobulin.

17. The method according to claim 16, **characterized in that** said sequence coding for the constant domain of the heavy chain preferentially consists in a sequence from an immunoglobulin with membrane anchoring, or comprising a transmembrane C-terminal region.

18. The method according to any one of claims 11 to 17, **characterized in that** it further comprises a step k) for transfecting with the vector obtained in step j) a host cell capable of expressing the heavy or light chain of the antibody coded by the double strand DNA fragment inserted within said vector.

19. The method according to claim 18, **characterized in that** said host cell of step k) is a cell capable of expressing at its surface the antibody coded by the inserted double strand DNA fragment.

20. The method according to claim 19, **characterized in that** said host cell is an eukaryotic cell.

21. The use of a method according to any one of claims 1 to 20 for generating a library of antibodies.

22. A method for *in vitro* screening of an active antibody against a given disease, **characterized in that** it applies a step in which the method according to any one of claims 18 to 20 is applied to a blood sample of a patient suffering from said given disease.

## Patentansprüche

1. Verfahren zur Erzeugung einer DNA-Sequenz, die für eine schwere Kette oder eine leichte Kette mindestens eines Antikörpers codiert, ausgehend von einem RNA-Extrakt oder einer RNA-Mischung, der bzw. die von einer Zelle abgeleitet ist, wobei diese Zelle fähig ist, RNA, die für einen Antikörper oder für die schwere Kette und/oder die leichte Kette eines Antikörpers codiert, zu exprimieren, **dadurch gekennzeichnet, dass** das Verfahren mindestens die folgenden Schritte umfasst:
a) Inkontaktbringen eines primären Primers (I) mit dem RNA-Extrakt oder der RNA-Mischung, der bzw. die geeignet ist, mindestens eine RNA, hier als "sense"-RNA bezeichnet, zu enthalten, die für die schwere Kette oder die leichte Kette eines Antikörpers codiert, wobei dieser primäre Primer spezifisch hybridisierbar ist mit einem Fragment der Sequenz der "sense"-RNA, wobei dieses Fragment in der Sequenz enthalten ist, die für die konstante Region C der schweren oder leichten Kette des Antikörpers codiert,
b) Synthetisieren einer einzelsträngigen cDNA, als "anti-sense"-cDNA bezeichnet, ausgehend von dem primären Primer (I);
c) gegebenenfalls Entfernen der primären Primer (I), die nicht mit dem Fragment der Sequenz der RNA in Schritt a) hydrisiert haben;
d) Zirkularisieren der "anti-sense"-cDNA durch Bildung einer kovalenten Bindung zwischen ihrem 5'- und 3'-Ende;
e) Inkontaktbringen eines sekundären Primers (II), als sekundärer "sense"-Primer bezeichnet, mit der in Schritt d) erhaltenen zirkulären "anti-sense"-cDNA, wobei dieser sekundäre "sense"-Primer (II) mit der zirkulären "anti-sense"-cDNA hybridisierbar ist;
f) Amplifizieren der "anti-sense"-cDNA ausgehend von dem sekundären Primer (II); und
g) Gewinnen des auf diese Weise amplifizierten komplementären linearen "sense"-DNA-Strangs.

2. Verfahren nach Anspruch 1, **dadurch gekennzeichnet, dass** der primäre Primer (I) spezifisch ist für eine Sequenz, die sich an, oder angrenzend an, dem 5'-Ende der "sense"-RNA-Sequenz, die der konstanten Region C entspricht, befindet.

3. Verfahren nach Anspruch 1, **dadurch gekennzeichnet, dass** der sekundäre Primer (II) spezifisch ist für die Sequenz zwischen dem 5'-Ende der "anti-sense"-cDNA-Sequenz und dem 3'-Ende der Sequenz der "anti-sense"-cDNA, die der konstanten Region C entspricht.

4. Verfahren nach Anspruch 3, **dadurch gekennzeichnet, dass** der sekundäre Primer (II) spezifisch ist für eine Sequenz, die sich an, oder angrenzend an, dem 5'-Ende der "anti-sense"-cDNA-Sequenz befindet.

5. Verfahren nach einem der Ansprüche 1 bis 4, **dadurch gekennzeichnet, dass** der primäre (I) und sekundäre (II) Primer jeweils und unabhängig voneinander von einer einzelsträngigen DNA-Sequenz mit einer Länge zwischen 10 und 100 Nukleotiden gebildet sind.

6. Verfahren nach einem der Ansprüche 1 bis 5, **dadurch gekennzeichnet, dass** es, vor dem Schritt a) des Hybridisierens des primären Primers (I), einen Schritt des Denaturierens der RNA umfasst.

7. Verfahren nach einem der Ansprüche 1 bis 6, **dadurch gekennzeichnet, dass** die Zelle, die fähig ist, einen Antikörper herzustellen, von einem Hybridom gebildet ist.

8. Verfahren nach einem der Ansprüche 1 bis 6, **dadurch gekennzeichnet, dass** die RNA ausgewählt ist aus der RNA, die von Splenozyten, Nodulen oder B-Lymphozyten abgeleitet ist.

9. Verfahren nach einem der Ansprüche 1 bis 8, **dadurch gekennzeichnet, dass** der Schritt des Zirkularisierens durch Inkontaktbringen mit einer Ligase durchgeführt wird, die fähig ist, durch kovalente Bindung die 5'- und 3'-Enden einer einzelsträngigen DNA zu verbinden, als "ssDNA-Ligase bezeichnet.

10. Verfahren zur Sequenzierung einer DNA-Sequenz, die für eine schwere Kette oder eine leichte Kette mindestens eines Antikörpers codiert, ausgehend von einem RNA-Extrakt oder einer RNA-Mischung, der bzw. die von einer Zelle abgeleitet ist, **dadurch gekennzeichnet, dass** es ein Verfahren zur Erzeugung einer DNA-Sequenz, die für eine schwere Kette oder eine leichte Kette mindestens eines Antikörpers codiert, nach einem der Ansprüche 1 bis 9 verwendet, und dass es ferner einen Schritt des Sequenzierens des linearen "sense"-DNA-Strangs, der in Schritt g) des Verfahrens zur Erzeugung der DNA-Sequenz gewonnen wurde, umfasst.

11. Verfahren zur Erzeugung einer DNA-Bank, die für mindestens eine schwere Kette oder eine leichte Kette mindestens eines Antikörpers codiert, ausgehend von einem RNA-Extrakt oder einer RNA-Mischung, der bzw. die von einer Zelle abgeleitet ist, **dadurch gekennzeichnet, dass** es ein Verfahren zur Erzeugung einer DNA-Sequenz, die für eine schwere Kette oder eine leichte Kette mindestens eines Antikörpers codiert, nach einem der Ansprüche 1 bis 10 verwendet, und dass es ferner die folgenden Schritte umfasst:
h) Inkontaktbringen eines tertiären Primers (III), als tertiärer "anti-sense"-Primer bezeichnet, mit der in Schritt g) erhaltenen linearen "sense"-DNA, wobei dieser tertiäre "anti-sense"-Primer (III) spezifisch hybridisierbar ist mit einem Fragment der Sequenz der linearen DNA, die sich zwischen dem 3'- und dem 5'-Ende des linearen DNA-Strangs befindet, und die der Sequenz dieser linearen DNA, die für die konstante Region C codiert, entspricht;
i) Erzeugen, ausgehend von dem tertiären Primer (III), eines Konkatemers durch Synthese des komplementären "anti-sense"-DNA-Strangs, und
j) Klonieren des auf dieses Weise erhaltenen doppelsträngigen DNA-Konkatemers in zuvor hergestellte Vektoren.

12. Verfahren nach Anspruch 11, **dadurch gekennzeichnet, dass** der tertiäre Primer (III) spezifisch ist für eine Sequenz, die sich an, oder angrenzend an, dem 5'-Ende der Sequenz der linearen "sense"-DNA, die der konstanten Region C entspricht, befindet.

13. Verfahren zum Erzeugen einer DNA-Bank, die für schwere oder leichte Ketten von Antikörpern codiert, nach Anspruch 11, **dadurch gekennzeichnet, dass** es vor dem Schritt j) einen Schritt umfasst, der darin besteht, das Konkatemer im Bereich der Sequenzen zu segmentieren, die den verwendeten Primern entsprechen.

14. Verfahren nach Anspruch 13, **dadurch gekennzeichnet, dass** der primäre Primer (I) eine Restriktionsstelle enthält.

15. Verfahren nach Anspruch 13 oder Anspruch 14, **dadurch gekennzeichnet, dass** der Schritt j) des Segmentierens des Konkatemers durch enzymatische Verdauung mit Hilfe von Restriktionsendonuclease durchgeführt wird, die spezifisch ist für die Restriktionsstelle, die in dem primären Primer (I) enthalten ist.

16. Vorrichtung nach einem der Ansprüche 11 bis 15, **dadurch gekennzeichnet, dass** der in Schritt j) verwendete Vektor ferner die Sequenzen enthält, die für die konstanten Domänen der schweren oder leichten Kette eines Immunoglobulins codieren.

17. Verfahren nach Anspruch 16, **dadurch gekennzeichnet, dass** die Sequenz, die für die konstante Domäne der schweren Kette codiert, vorzugsweise von einer Sequenz gebildet ist, die von einem Immunoglobin mit Membrananker abgeleitet ist oder eine transmembrane C-terminale Region enthält.

18. Vorrichtung nach einem der Ansprüche 11 bis 17, **dadurch gekennzeichnet, dass** es ferner einen Schritt k) der Transfektion einer Wirtszelle mit dem in Schritt j) erhaltenen Vektor umfasst, wobei die Wirtszelle fähig ist, die schwere oder leichte Kette des Antikörpers zu exprimieren, der durch das doppelsträngige DNA-Fragment, das in den Vektor integriert wurde, codiert wird.

19. Verfahren nach Anspruch 18, **dadurch gekennzeichnet, dass** die Wirtszelle in Schritt k) eine Zelle ist, die fähig ist, an ihrer Oberfläche den Antikörper zu exprimieren, der durch das integrierte doppelsträngige DNA-Fragment codiert wird.

20. Verfahren nach Anspruch 19, **dadurch gekennzeichnet, dass** die Wirtszelle eine Eukaryotenzelle ist.

21. Verwendung eines Verfahrens nach einem der Ansprüche 1 bis 20 für die Erzeugung einer Antikörperbibliothek.

22. Verfahren zum in-vitro-Screening von Antikörpern, die gegen eine bestimmte Krankheit wirken, **dadurch gekennzeichnet, dass** es einen Schritt verwendet, in dem das Verfahren nach einem der Ansprüche 18 bis 20 auf eine Blutprobe angewendet wird, die zuvor einem Patienten, der an der bestimmten Krankheit leidet, entnommen wurde.


### SEQUENCE LISTING

<110> Pierre Fabre Médicament
<120> Nouveau procédé de génération et de criblage d'une banque d'anticorps
<130> D25994
<150> FR0708444
   <151> 2007-12-04
<160> 41
<170> Patent In version 3. 3
<210> 1
   <211> 29
   <212> DNA
   <213> Séquence artificielle
<220>
   <223> Amorce I chaîne lourde
<400> 1
   agcagacccg ggggccagtg gatagacag 29
<210> 2
   <211> 44
   <212> DNA
   <213> Séquence artificielle
<220>
   <223> Amorce I chaîne légère
<400> 2
   t ccagat gt t aact gct cac t ggat ggt gg gaagat ggat acag 44
<210> 3
   <211> 20
   <212> DNA
   <213> Séquence artificielle
<220>
   <223> Armorce II chaîne lourde
<400> 3
   cgt ct gggcc cccggt cacc 20
<210> 4
   <211> 19
   <212> DNA
   <213> Séquence artificielle
<220>
   <223> Amorce II chaîne légère
<400> 4
   ggt ct acaat t gacgagt g 19
<210> 5
   <211> 21
   <212> DNA
   <213> Séquence artificielle
<220>
   <223> Amorce III chaîne lourde
<400> 5
   gat agacaga t gggggt gt c g 21
<210> 6
   <211> 19
   <212> DNA
   <213> Séquence artificielle
<220>
   <223> Amorce III chaîne légère
<400> 6
   ggt gggaaga t ggat acag 19
<210> 7
   <211> 411
   <212> DNA
   <213> Séquence artificielle
<220>
   <223> Domaine variable chaîne lourde (selon l'invention)
<400> 7
   atggaatgga gctggatctt tctctttctc ctgtcaggaa ctgcaggtgt cctctctgag 60
   gtccagctgc aacagtttgg agctgagctg gtgaagcctg gggcttcagt gaagatatcc 120
   tgcaaggctt ctggctacac attcactgac tacaacatgg actgggtgaa gcagagccat 180
   ggaaagagcc ttgagtggat tggagatatt aatcctaact atgatcgtac tacctacaac 240
   cagaagttca agggaaaggc cacattgact gtagacaagt cctccagcac agcctacatg 300
   gagctccgca gcctgacatc tgaggacact gcagtctatt actgtgcaag aagggagatt 360
   acgacggaat ttgactactg gggccaaggc accactctca cagtctcctc a 411
<210> 8
   <211> 411
   <212> DNA
   <213> Séquence artificielle
<220>
   <223> Domaine variable chaîne lourde (approche classique)
<400> 8
   atgggatgga gcggggtctt tatctttctc ctgtcaggaa ctgcaggtgt cctctctgag 60
   gtccagctgc aacagtttgg agctgagctg gtgaagcctg gggcttcagt gaagatatcc 120
   tgcaaggctt ctggctacac attcactgac tacaacatgg actgggtgaa gcagagccat 180
   ggaaagagcc ttgagtggat tggagatatt aatcctaact atgatcgtac tacctacaac 240
   cagaagttca agggaaaggc cacattgact gtagacaagt cctccagcac agcctacatg 300
   gagctccgca gcctgacatc tgaggacact gcagtctatt actgtgcaag aagggagatt 360
   acgacggaat ttgactactg gggccaaggc accactctca cagtctcctc a 411
<210> 9
   <211> 57
   <212> DNA
   <213> Séquence artificielle
<220>
   <223> Séquence nucléique codant pour le peptide signal de la chaîne lourde (selon l'invention)
<400> 9
   at ggaat gga gct ggat ct t ct ctttctc ct gt caggaa ct gcaggt gt cct ct ct 57
<210> 10
   <211> 57
   <212> DNA
   <213> Séquence artificielle
<220>
   <223> Séquence nucléique codant pour le peptide signal de la chaîne lourde (approche classique)
<400> 10
   at gggat gga gcggggt ct t tat ct t t ct c ct gt caggaa ct gcaggt gt cct ct ct 57
<210> 11
   <211> 541
   <212> DNA
   <213> Séquence artificielle
<220>
   <223> Séquence nucléique codant pour la chaîne lourde totale
<400> 11
   t ct agaggat ccccgggt ct gct aaaaat a t gt ccaat gt cct ct ccaca gacact gaac 60
   acact gact c t aaccat gga at ggagct gg at ct t t ct ct t t ct cct gt c aggaact gca 120
   ggt gt cct ct ct gaggt cca gct gcaacag t t t ggagct g agct ggt gaa gcct ggggct 180
   t cagt gaaga t at cct gcaa ggct t ct ggc t acacat t ca ct gact acaa cat ggact gg 240
   gt gaagcaga gccat ggaaa gagcct t gag t ggat t ggag at at t aat cc t aact at gat 300
   cgtactacct acaaccagaa gttcaaggga aaggccacat tgactgtaga caagtcctcc    360
   agcacagcct acatggagct ccgcagcctg acatctgagg acactgcagt ctattactgt    420
   gcaagaaggg agattacgac ggaatttgac tactggggcc aaggcaccac tctcacagtc    480
   tcctcagcca aacgacaccc cccatctgtc tatccactgg cccccgggat accgagctcg    540
   a 541
<210> 12
   <211> 522
   <212> DNA
   <213> Séquence artificielle
<220>
   <223> Séquence codant pour la chaîne légère totale (selon l'invention)
<400> 12
   cggccgcact agtgataaca tcttaagcat cctctcttcc agctctcaga gatggagaca     60
   gacacact cc t gct at gggt gct gct gct c t gggt t ccag gt t ccacagg t gacat t gt g 120
   ttgacccaat ctccagcttc tttggctgtg tctctagggc agagggccac catatcctgc    180
   agagccagt g aaaat gt t ga t agt t at ggc aat agt t t t a t gcact ggt a t cagcagaaa 240
   ccaggacagc cacccaaact cctcatctat cgtgcatcca acctagaatc tgggatccct    300
   gccaggt t ca gt ggcagt gg gt ct aggaca gact t caccc t caccat t aa t cct gt ggag 360
   gct gat gat g t t gcaacct a t t act gt caa caaagt aat g aggat ccgt a cacgt t cgga 420
   ggggggacca agctggaaat aaaacgggct gatgctgcac caactgtatc catcttccca    480
   ccatccagtg agcagttatc ccgcggccat ggcggccggg ag                       522
<210> 13
   <211> 333
   <212> DNA
   <213> Séquence artificielle
<220>
   <223> Domaine variable chaîne légère (selon invention)
<400> 13
   gacattgtgt tgacccaatc tccagcttct ttggctgtgt ctctagggca gagggccacc    60
   at at cct gca gagccagt ga aaat gt t gat agt t at ggca at agt t t t at gcact ggt at 120
   cagcagaaac caggacagcc acccaaactc ctcatctatc gtgcatccaa cctagaatct   180
   gggat ccct g ccaggt t cag t ggcagt ggg t ct aggacag act t caccct caccat t aat 240
   cct gt ggagg ct gat gat gt t gcaacct at t act gt caac aaagt aat ga ggat ccgt ac 300
   acgttcggag gggggaccaa gctggaaata aaa                                333
<210> 14
   <211> 31
   <212> DNA
   <213> Séquence artificielle
<220>
   <223> Amorce primaire chaîne lourde
<400> 14
   acaaacgcgt atagcccttg accaggcat c c 31
<210> 15
   <211> 30
   <212> DNA
   <213> Séquence artificielle
<220>
   <223> Amorce primaire chaîne légère
<400> 15
   acaaacgcgt t ggt gggaag at ggat acag 30
<210> 16
   <211> 20
   <212> DNA
   <213> Séquence artificielle
<220>
   <223> Armorce secondaire chaîne lourde
<400> 16
   ct aact ccat ggt gaccct g 20
<210> 17
   <211> 19
   <212> DNA
   <213> Séquence artificielle
<220>
   <223> Armorce secondaire chaîne légère
<400> 17
   gggct gat gc tgcaccaac 19
<210> 18
   <211> 576
   <212> DNA
   <213> Séquence artificielle
<220>
   <223> Séquence codant pour la chaîne lourde totale (selon l'invention
<400> 18
   acgcgt t t gt acaacat at g t ccaat gt cc t ct cct caga cact gaacac act gact ct a 60
   accat gggat ggagct ggat ct t t ct ct t t ct cct gt cag gaact gcagg t gt cct ct ct 120
   gaggt ccagc t gcaacagt c t ggacct gag ct ggt gaagc ct ggggct t c agt gaagat a 180
   tcctgcaaga cttctggata catattcact gcatacacca tgcactgggt gaggcagagc 240
   ct t ggagaga gcct t gact g gat t ggaggt at t aaaccaa acaat ggt ct t gct aact ac 300
   aaccagaagt tcaagggcaa ggccacattg actgtagaca agtcctccag cacagcctac 360
   at ggacct cc gcagcct gac at ct gaggat t ct gcagt ct at t act gt gc aagat ct gag 420
   at t acgacgg aat t t gact a ct ggggccaa ggcaccgct c t cacagt ct c ct cagccaaa 480
   acgacacccc catctgtcta tccactggcc cctggatctg ctgcccaaac taactccatg 540
   gt gaccct gg gat gcct ggt caagggct at acgcgt 576
<210> 19
   <211> 354
   <212> DNA
   <213> Séquence artificielle
<220>
   <223> Domaine variable chaîne lourde ( sel on l'invention)
<400> 19
   gaggt ccagc t gcaacagt c t ggacct gag ct ggt gaagc ct ggggct t c agt gaagat a 60
   tcctgcaaga cttctggata catattcact gcatacacca tgcactgggt gaggcagagc 120
   ct t ggagaga gcct t gact g gat t ggaggt at t aaaccaa acaat ggt ct t gct aact ac 180
   aaccagaagt tcaagggcaa ggccacattg actgtagaca agtcctccag cacagcctac 240
   at ggacct cc gcagcct gac at ct gaggat t ct gcagt ct at t act gt gc aagat ct gag 300
   at t acgacgg aat t t gact a ct ggggccaa ggcaccgct c t cacagt ct c ct ca 354
<210> 20
   <211> 521
   <212> DNA
   <213> Séquence artificielle
<220>
   <223> Séquence codant pour la chaîne lourde totale (selon l'invention)
<400> 20
   cat at gt cca at gt cct ct c ct cagacact gaacacact g act ct aacca t gggat ggag 60
   ct ggat ct t t ct ct t t ct cc t gt caggaac t gcaggt gt c ct ct ct gagg t ccagct gca 120
   acagt ct gga cct gagct gg t gaagcct gg ggct t cagt g aagat at cct gcaagact t c 180
   tggatacata ttcactgcat acaccatgca ctgggtgagg cagagccttg gagagagcct    240
   tgactggatt ggaggtatta aaccaaacaa tggtcttgct aactacaacc agaagttcaa    300
   gggcaaggcc acattgactg tagacaagtc ctccagcaca gcctacatgg acctccgcag    360
   cct gacat ct gaggat t ct g cagt ct at t a ct gt gcaaga t ct gagat t a cgacggaat t 420
   tgactactgg ggccaaggca ccgctctcac agtctcctca gccaaaacga cacccccatc    480
   t gt ct at cca ct ggcccct g gat ct gct gc ccaaact aac t 521
<210> 21
   <211> 354
   <212> DNA
   <213> Séquence artificielle
<220>
   <223> Domaine variable chaîne lourde (selon l'invention)
<400> 21
   gaggt ccagc t gcaacagt c t ggacct gag ct ggt gaagc ct ggggct t c agt gaagat a 60
   tcctgcaaga cttctggata catattcact gcatacacca tgcactgggt gaggcagagc    120
   ct t ggagaga gcct t gact g gat t ggaggt at t aaaccaa acaat ggt ct t gct aact ac 180
   aaccagaagt tcaagggcaa ggccacattg actgtagaca agtcctccag cacagcctac    240
   at ggacct cc gcagcct gac at ct gaggat t ct gcagt ct at t act gt gc aagat ct gag 300
   at t acgacgg aat t t gact a ct ggggccaa ggcaccgct c t cacagt ct c ct ca 354
<210> 22
   <211> 31
   <212> DNA
   <213> Séquence artificielle
<220>
   <223> Amorce primaire murine
<400> 22
   acaaacgcgt at aaccct t g accaggcat c c 31
<210> 23
   <211> 30
   <212> DNA
   <213> Séquence artificielle
<220>
   <223> Amorce primaire murine
<400> 23
   acaaacgcgt cagggat cca gagt t ccaag 30
<210> 24
   <211> 31
   <212> DNA
   <213> Séquence artificielle
<220>
   <223> Armorce primaire murine
<400> 24
   acaaacgcgt gt agcct t t g acaaggcat c c 31
<210> 25
   <211> 19
   <212> DNA
   <213> Séquence artificielle
<220>
   <223> Amorce secondaire murine
<400> 25
   act ggct cct cggt gact c 19
<210> 26
   <211> 21
   <212> DNA
   <213> Séquence artificielle
<220>
   <223> Amorce secondaire murine
<400> 26
   agt gact gt g act t ggaact c 21
<210> 27
   <211> 19
   <212> DNA
   <213> Séquence artificielle
<220>
   <223> Amorce secondaire murine
<400> 27
   gt ct at ccac tggcccctg 19
<210> 28
   <211> 20
   <212> DNA
   <213> Séquence artificielle
<220>
   <223> Amorce secondaire murine
<400> 28
   ct ggat cct c ggt gacact g 20
<210> 29
   <211> 18
   <212> DNA
   <213> Séquence articielle
<220>
   <223> Armorce tertiaire murine
<400> 29
   gat agaccga t ggggct g 18
<210> 30
   <211> 21
   <212> DNA
   <213> Séquence artificielle
<220>
   <223> Amorce tertiaire murine
<400> 30
   gat agact ga t gggggt gt t g 21
<210> 31
   <211> 18
   <212> DNA
   <213> Séquence artificielle
<220>
   <223> Amorce tertiaire murine
<400> 31
   gat agacaga t ggggct g 18
<210> 32
   <211> 24
   <212> DNA
   <213> Séquence artificielle
<220>
   <223> Amorce primaire humaine
<400> 32
   acgcgtttga ccaggcagcc cagg 24
<210> 33
   <211> 31
   <212> DNA
   <213> Séquence artificielle
<220>
   <223> Amorce primaire humaine
<400> 33
   acgcgt caga t t t caact gc t cat cagat g g 31
<210> 34
   <211> 26
   <212> DNA
   <213> Séquence artificielle
<220>
   <223> Amorce primaire humaine
<400> 34
   acgcgt agt g t ggcct t gt t ggct t g 26
<210> 35
   <211> 18
   <212> DNA
   <213> Séquence artificielle
<220>
   <223> Amorce secondaire humaine
<400> 35
   gccct gggct gcctggtc 18
<210> 36
   <211> 25
   <212> DNA
   <213> Séquence artificielle
<220>
   <223> Amorce secondaire humaine
<400> 36
   cat ct gat ga gcagt t gaaa t ct gg 25
<210> 37
   <211> 19
   <212> DNA
   <213> Séquence artificielle
<220>
   <223> Amorce secondaire humaine
<400> 37
   caagccaaca aggccacac 19
<210> 38
   <211> 16
   <212> DNA
   <213> Séquence artificielle
<220>
   <223> Amorce tertiaire humaine
<400> 38
   gat gggccct t ggt gg 16
<210> 39
   <211> 22
   <212> DNA
   <213> Séquence artificielle
<220>
   <223> Amorce tertiaire humaine
<400> 39
   ggaagat gaa gacagat ggt gc 22
<210> 40
   <211> 21
   <212> DNA
   <213> Séquence artificielle
<220>
   <223> Amorce tertiaire humaine
<400> 40
   ggagggyggg aacagagtga c 21
<210> 41
   <211> 19
   <212> DNA
   <213> Séquence artificielle
<220>
   <223> Amorce tertiaire murine
<400> 41
   cagt t ggt gc agcat cagc 19